(19) Europäisches Patentamt
European Patent Office
Office européen des brevets

(11) **EP 2 310 059 B1**

(12) **EUROPEAN PATENT SPECIFICATION**

(45) Date of publication and mention
of the grant of the patent:
**16.11.2016  Bulletin 2016/46**

(21) Application number: **09753968.8**

(22) Date of filing: **29.05.2009**

(51) Int Cl.:
*A61L 27/06* (2006.01)  *A61L 27/34* (2006.01)
*A61L 27/50* (2006.01)  *A61L 27/28* (2006.01)
*A61L 31/02* (2006.01)  *A61L 31/08* (2006.01)
*A61L 31/14* (2006.01)

(86) International application number:
**PCT/EP2009/056666**

(87) International publication number:
**WO 2009/144313 (03.12.2009 Gazette 2009/49)**

(54) **PUFA COVERED IMPLANTS**

MIT PUFA BESCHICHTETE IMPLANTATE

IMPLANTS RECOUVERTS D'ACIDE GRAS POLYINSATURÉ

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **29.05.2008  US 56978**

(43) Date of publication of application:
**20.04.2011  Bulletin 2011/16**

(73) Proprietor: **Numat Biomedical S.L.**
**07121 Palma de Mallorca (ES)**

(72) Inventors:
• **LYNGSTADAAS, Ståle Petter**
**N-1450 Nesoddtangen (NO)**
• **MONJO, Marta**
**E-07014 Palma de Mallorca (ES)**
• **PETZOLD, Christiane**
**0272 Oslo (NO)**

• **ELLINGSEN, Jan Eirik**
**N-1356 Bekkestau (NO)**

(74) Representative: **ZBM Patents - Zea, Barlocci & Markvardsen**
**Plaza Catalunya, 1**
**08002 Barcelona (ES)**

(56) References cited:
**US-A1- 2006 088 596    US-A1- 2007 202 149**
**US-A1- 2007 299 512    US-A1- 2008 038 307**
**US-A1- 2008 118 544**

• **PAKALA RAJBABU ET AL: "Eicosapentaenoic acid and docosahexaenoic acid block serotonin-induced smooth muscle cell proliferation" ARTERIOSCLEROSIS, THROMBOSIS, AND VASCULAR BIOLOGY, LIPPINCOTT WILLIAMS & WILKINS, US, vol. 19, no. 10, 1 October 1999 (1999-10-01), pages 2316-2322, XP002272097 ISSN: 1079-5642**

**Description**

*Field of the invention*

The present invention relates to a coated metal implant to be used as medical and/or dental implant, which actively facilitates controlled adhesion of hard and/or mineralized tissue to the implant due to at least part of its surface being coated with a low concentration layer of polyunsaturated fatty acids (PUFA) and irradiated with UV light, before and/or after the coating.

*Background of the invention*

Medical implants, such as dental implants, orthopaedic implants, prosthesis and vascular stents are commonly made of titanium and/or a titanium alloy. Titanium is the material most frequently used as implant in bone, as it has outstanding physical and biological properties, such as low density, mechanical strength, and chemical resistance against body fluids.

Dental implants are utilized in dental restoration procedures in patients having lost one or more of their teeth. A dental implant comprises a dental fixture, which is utilized as an artificial tooth root replacement. Thus, the dental fixture serves as a root for a new tooth. Typically, the dental fixture is a titanium screw which has a roughened surface in order to expand the area of tissue contact. The titanium screw is surgically implanted into the jawbone, where after the bone tissue grows around the screw. This process is called osseointegration, because osteoblasts grow on and into the rough surface of the implanted screw. By means of osseointegration, a rigid installation of the screw is obtained.

Once the titanium screw is firmly anchored in the jawbone, it may be prolonged by attachment of an abutment to the screw. The abutment may, just as the screw, be made of titanium or a titanium alloy. The shape and size of the utilized abutment are adjusted such that it precisely reaches up through the gingiva after attachment to the screw. A dental restoration such as a crown, bridge or denture may then be attached to the abutment. Alternatively, the titanium screw has such a shape and size that it reaches up through the gingiva after implantation, whereby no abutment is needed and a dental restoration such as a crown, bridge or denture may be attached directly to the screw.

Orthopedic implants are utilized for the preservation and restoration of the function in the musculoskeletal system, particularly joints and bones, including alleviation of pain in these structures. Vascular stents are tubular implants arranged for insertion into blood vessels in order to prevent or counteract a localized flow constriction, i.e. they counteract significant decreases in blood vessel diameter.

As already mentioned above, titanium (Ti) is the implant material of choice for use in dental and orthopaedic applications and in vascular stents. The stable oxides that form readily on Ti surfaces have been reported to attribute to its excellent biocompatibility. However, it has also been reported that bone response to implant surfaces was dependent on the chemical and physical properties of Ti surfaces, thereby affecting implant success. As such, attention has been focused on the surface preparation of Ti implants.

The surface of Ti is only bioinert, thus current research on modification of implant surfaces focuses on making virtual bioinert materials become bioactive, or rather to influence the types of proteins absorbed at the surface readily after implantation. The assortment of surface modifications ranges from non-biological coatings, such as carbide, fluorine, calcium, hydroxyl apatite or calcium phosphate, to coatings that are to mimic the biological surroundings using lipid mono- or bi-layers, growth factors, proteins, and/or peptides.

Coatings for implantable medical devices containing unsaturated fatty acids have been disclosed in the state of the art. Particularly, the United States patent applications US2006088596, US2007202149 and US2008118544 disclose coatings that comprise polyunsaturated fatty acids such as eicosapentaenoic acid (EPA) and docosahexanoic acid (DHA) and vitamin E. It is also disclosed a process for the preparation of those coated medical devices that comprises exposing the coating to UV light.

On the other hand, the United States patent application US2007299512 discloses an implant having a coating containing cholesterol and cholesterol esters that can be additionally coated by polyunsaturated fatty acids or vitamins. And the United States patent application US2008118544 disclose hemocompatible surfaces of medical products coated with unsaturated fatty acids that are prepared by exposing the the coating to UV light.

E.g. several techniques, such as plasma spraying, laser deposition, ion beam dynamic mixing, ion beam deposition, magnetic sputtering, hot isostatic pressing, electrophoretic deposition, sol-gel, ion implantation, NaOH treatment, and electrochemical methods have been employed to deposit hydroxyapatite (HA) or calcium phosphate coatings on Ti surfaces.

It has been reported that implants coated with hydroxyapatite (HA) enhances osteoinduction. The superior performance of these implants being attributed to more rapid osseointegration and the development of increased interfacial strength, which results from early skeletal attachment and increased bone contact with the implant's surface.

Especially plasma spraying has been employed frequently, however with numerous problems, including variation in bond strength between the coating and the metallic substaret, non-uniformity in the layer thickness, and poor adhesion between the coating and the metal surface (Satsangi et al., 2004).

It has also been proposed to improve the biocompatibility of prostheses or implants by binding or integrating various active biomolecules to the surface of the prosthesis, e.g. on to the metallic surface of a titanium prosthesis. It has been the aim with implants prepared this way that they have improved fit; exhibit increased tissue stickiness and increased tissue compatibility; have a biologically active surface for increased cell growth, differentiation and maturation; exhibit reduced immunoreactivity; exhibit antimicrobial activity; exhibit increased biomineralisation capabilities; result in improved wound and/or bone healing; lead to improved bone density; have reduced "time to load" and cause less inflammation. Such binding has often been proposed carried out using for example chemical reactants having two reactive functionalities such as formalin or glutaraldehyde, but the reactive nature of these agents often leads to the biomolecules becoming biologically inactive and/or with enhanced immunoreactivity, which is of course undesirable.

An alternative surface modification is using phospholipids coating which is reported to induce the deposition of calcium phosphate. The role of phospholipids has also been suggested in the initiation of calcium phosphate deposition in cartilage, bone, healing fracture callus and calcifying bacteria. It has been proposed that an implant surface coated with a calcium-phospholipid-phosphate should be able to attract hydroxyapaptite.

Surface coatings of lipid mono- or bilayers are presumed to mimic cell surfaces and therewith to prevent foreign body reactions. Lipid coatings have been shown to influence the attachment of proteins to a surface that occurs immediately after the implantation and were found to prevent cell adhesion and blood clot formation (Kim et al., 2005). Certain phospholipids were furthermore reported to decrease bacterial adhesion.

Lipid coatings are usually based on physical adhesion, where ordered layers are obtained e.g. by using Langmuir-Blodgett technique. Lipids of the cell membrane are not only forming passive surroundings for the proteins incorporated into the membranes but rather influence the cell metabolism actively. Chemical methods for coating of metal substrates with a layer of biological molecules usually involve a foregoing step for obtaining reactive groups on surfaces (Khan W et al., 2007; Muller R et al., 2006) in order to bind the biologically active molecules without altering their structure and therewith possibly their function in the body.

Still, the coatings mentioned above all struggle from several draw-backs, due to unresolved technical difficulties.

*Summary of the invention*

The present invention for the first time describes a coated metal implant to be used as medical and/or dental implant, which actively facilitates control of hard and/or mineralized tissue adhesion to the implant, such as bone, cartilage or dentin addition to the implant surface.

A typical implant of the present invention either actively facilitates controlling hard and/or mineralized tissue adhesion to the implant, controlling bone addition to the implant surface, improved bone remodeling and/or biocompatibility of the implant. The effect of the implant on mineralized and/or hard tissue adhesion being directly attributable to at least part of its surface being coated with a low, or with a high concentration layer of polyunsaturated fatty acids (PUFA).

Thus, the invention relates to a coated metal implant for controlled adhesion of mineralized and/or hard tissue, wherein at least part of the surface of the implant is coated with chemically UV-bound PUFA (polyunsaturated fatty acids) at a concentration of 10 nanogram/mm$^2$, or less, wherein the coated metal implant is obtainable by a method comprising: i) mirror polishing the implant, ii) washing, iii) autoclaving, a) treating the implant with a solution comprising PUFA, and b) irradiating at least part of the surface of the implant with UV of 100 to 315 nm for a period of time comprised from 30 seconds to 30 minuts and optionally c) washing said implant, wherein step a) and b) are performed simultaneously or in any order.

The present invention at the same time discloses a metal implant with improved biocompatibility which can facilitate a solid incorporation into the bone, and to an implant which is easy to remove again, wherein the high concentration of available double bounds from the polyunsaturated fatty acids hinder tissue adherence to a semi-permanent and/or temporary implant, a so called "slippery" implant.

The invention further discloses a method for manufacturing said metal implant with controlled effect on hard and/or mineralized tissue adhesion and/or bone remodeling, wherein the implant is coated with PUFA at a specific concentration and irradiated with UV light, either before, simultaneously with, or after the coating step.

The invention consequently relates to a novel and simple surface modification method to chemically bind PUFA molecules to a surface comprising Ti or a titanium oxide by utilizing UV irradiation. A method is thus presented for manufacturing a metal implant which facilitates controlled hard and/or mineralized tissue adhesion, controlled bone addition, improved effect on bone remodeling and/or biocompatibility, wherein the implant is coated with PUFA at a specific concentration and irradiated with UV light, before and/or after the coating.

Objects and features of the present invention will become apparent from the claims and the following detailed description considered in conjunction with the accompanying drawings. It is to be understood, however, that the drawings are designed solely for purposes of illustration and not as a definition of the limits of the invention, for which reference should be made to the appended claims.

*Figure legends*

Figure 1: Modification of the 3 groups of coin surfaces. Each group contains a control with no EPA on the surface, and surfaces with 3.2 nmol EPA/mm$^2$ or 16.3 nmol EPA/mm$^2$ given to the respective surfaces.

Figure 2: Water contact angle against UV irradiation time (average for 3 surfaces). After a slight increase for about 10 min of irradiation the water CA decreased. The black point indicates the contact angle after 30 min, where a decrease was found for all the surfaces. After 60 min the decrease of the water CA flattened.

Figure 3: Contact angles measured on coins of the 3 different groups with different concentrations of EPA solutions used for surface modification. A - non-irradiated, unwashed surfaces with a thick layer of physically bound EPA; B - non-irradiated, washed surfaces with a thin layer of physically adsorbed EPA; C - irradiated, washed surfaces with a thin layer of covalently bound EPA.

Figure 4: Profilometric observation of EPA drops on polished Ti surfaces. 3.2 nmol EPA/mm$^2$ was given to the surface and pictures were taken a) before UV irradiation, and b) after 30 min irradiation with UV light. The pictures were taken with 50x magnification.

Figure 5: Changes in the FTIR spectra of EPA and EPA after irradiation for 30 min. The numbers from 1 to 8 highlight the main changes in the structure as referred to in the text.

Figure 6: Results for scintillation countings of $^{14}$C-EPA coated coins which were either non-irradiated or irradiated with UV light for 30 min. The coins were unwashed, washed once, and washed twice, respectively.

Figure 7: Amount of cells on the surfaces after 24 h relative to plastic (100%) as calculated from the amount of DNA on the respective surfaces. A - non-irradiated, unwashed surfaces with a thick layer of physically bound EPA; B - non-irradiated, washed surfaces with a thin layer of physically adsorbed EPA; C - irradiated, washed surfaces with a thin layer of covalently bound EPA.

Figure 8: Toxicity of surface modifications relative to plastic (0% toxicity) and 0.1% Triton X-100 (100% toxicity) as measured by LDH-level on the respective surfaces. A- non-irradiated, unwashed surfaces with a thick layer of physically bound EPA; B - non-irradiated, washed surfaces with a thin layer of physically adsorbed EPA; C - irradiated, washed surfaces with a thin layer of covalently bound EPA.

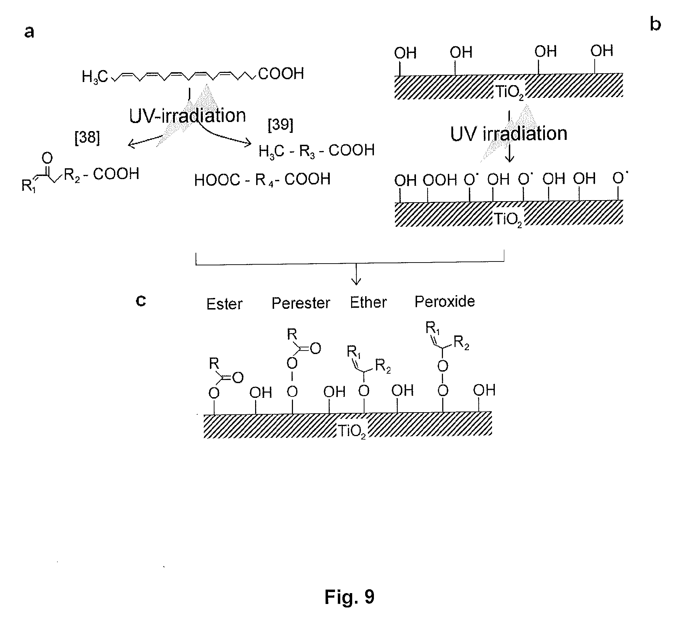

Figure 9: Proposed mechanism for the photooxidation of EPA and TiO$_2$ which resulted in a thin layer of covalently bound EPA and EPA photooxidation products. a) EPA is oxidized resulting in generation of oxides and their radicals, peroxides and their radicals, peroxy acids, and peresters within the EPA structure by irradiation with UV light, and degradation of EPA molecules into carboxylic and dicarboxylic acids; b) photooxidation of TiO$_2$ surfaces led to hydroxidation as well as reduction of hydroxide groups; c) bonding between reactive EPA-photooxidation products and reactive groups on the irradiated TiO$_2$ surfaces via formation of ester, perester, ether, and peroxide groups. R1-4 - remaining EPA backbone of various length and with various numbers of double bonds.

Figure 10. Pull-out test measurements of tested surfaces after 10 weeks healing period. Boxplots represent the median value and the distribution of the different measurements (n=6) of each group.

Figure 11. Changes in volumetric bone mineral density (vBMD) of sub-implant cortical bone after 10 weeks of healing time using micro-CT. Values represent means ± SEM (n=6).

Figure 12. LDH activity measured in the wound fluid collected from the implant site after 10 weeks of healing time.

Figure 13. ALP activity measured in the wound fluid collected from the implant site after 10 weeks of healing time.

Figure 14. Total protein measured in the wound fluid collected from the implant site after 10 weeks of healing time.

Figure 15. Osteocalcin gene expression in the peri-implant bone tissue attached to the modified titanium implants.

Figure 16. IL-6 gene expression in the peri-implant bone tissue attached to the modified titanium implants.

Figure 17. TRAP gene expression in the peri-implant bone tissue attached to the modified titanium implants.

Figure 18. Changes of peak areas of different absorbances in the FTIR spectrum caused by UV irradiation of PUFA.

Figure 19. α-tocopherol and some of its possible photooxidation products (after Yamauchi et al, 2002).

Figure 20. Change of peak areas at typical absorbances for α-tocopherol during UV irradiation.

Figure 21. Changes of peak areas with UV irradiation time when 50 mol% of α-tocopherol was added to EPA or DHA illustrating the antioxidative effect of α-tocopherol on PUFAs.

Figure 22. Changes of peak areas with a maximum absorbance at 3300 cm-1 for PUFAs with different amounts of α-tocopherol added.

Figure 23. Scheme over the transformation from 7DHC to 1,25(OH)$_2$D$_3$.

Figure 24. FTIR spectra for 7-DHC that was irradiated with UV light for different periods of time.

Figure 25. FTIR spectra for cholecalciferol that was irradiated with UV light for different periods of time.

Figure 26. Changes of peak areas with UV irradiation time for the absorbance spectra of 7-DHC and cholecalciferol.

Figure 27. The spectra of the mixtures of 7-DHC+EPA on Ti surfaces before and after UV irradiation for 15, 30, and 60 min.

Figure 28. Changes in peak areas of the mixture of 7-DHC + EPA on Ti surfaces during UV irradiation for up to 60 min compared to the changes of peak areas of EPA alone and 7-DHC alone.

Figure 29. Set-up for irradiating Ti samples with UV light after the application of mixtures of PUFA and/or vitamins.

## *Detailed description*

The present invention for the first time describes a novel and simple surface modification method to chemically bind polyunsaturated fatty acids (PUFA) molecules to a metal, such as to a surface comprising Ti and/or a titaniumoxide by utilizing UV irradiation.

In a particular embodiment, the PUFA used is eicosapentaenoic acid 20:5 n-3 (EPA), which is a fatty acid with positive effects on bone homeostasis *in vivo,* such as on bone remodeling, bone formation and/or bone resorption. UV light is herein employed to induce reactive binding sites on at least part of one of the Ti surfaces of a medical implant (photocatalytic effect) and/or within the EPA molecules, thus the UV radiation of the implant is envisioned either to take place before and/or after the coating of the Ti surface with PUFA.

As is documented in the experimental section (see e.g. Example 2), the surface coating of an implant thus coated and exposed to UV irradiation (chemically bound PUFA) has been characterized by CA measurement, FTIR and scintillation counting. Performance of the coated surfaces have further been tested *in vitro* with cultivation of MC3T3-E1 cells for 24 h and been compared to surfaces with physically adsorbed EPA (i.e. physically adsorbed PUFA) or to non-modified Ti surfaces, as well as to Ti surfaces that were exposed to UV irradiation without a pre-coating with PUFA. Surprisingly, surfaces with chemically bound EPA were shown to perform significantly better in terms of cell attachment compared to non-modified or only UV irradiated Ti surfaces as well as compared to surfaces with physically adsorbed EPA. What is more, they even exhibited a comparably low toxicity.

In the present context, the phrase "implant" includes within its scope any device intended to be implanted into the body of a vertebrate animal, in particular a mammal such as a human. Non-limiting examples of such devices are medical devices that replace anatomy or restore a function of the body such as the femoral hip joint; the femoral head; acetabuiar cup; vascular stents, elbow including stems, wedges, articular inserts; knee, including the femoral and tibial components, stem, wedges, articular inserts or patellar components; shoulders including stem and head; wrist; ankles; hand; fingers; toes; vertebrae; spinal discs; artificial joints; dental implants; ossiculoplastic implants; middle ear implants including incus, malleus, stages, incus-stapes, malleus-incus, malleus- incus-stapes; cochlear implants; orthopacdic fixation devices such as nails, screws, staples and plates; heart valves; pacemakers; catheters; vessels; space filling implants; implants for retention of hearing aids; implants for external fixation; and also intrauterine devices (IUDs); and bioelectronic devices such as intracochlear or intracranial electronic devices. Medical implants may also be denoted as medical prosthetic devices. Generally, a medical implant is composed of one or several implant parts.

In the present context, the term "orthopedic implant" includes within its scope any device intended to be implanted into the body of a vertebrate animal, in particular a mammal such as a human, for preservation and restoration of the function of the musculoskeletal system, particularly joints and bones, including the alleviation of pain in these structures.

In the present context, the term "dental implant" includes within its scope any device intended to be implanted into the oral cavity of a vertebrate animal, in particular a mammal such as a human, in tooth restoration procedures. Dental implants may also be denoted as dental prosthetic devices. Generally, a dental implant is composed of one or several implant parts. For instance, a dental implant usually comprises a dental fixture coupled to secondary implant parts, such as an abutment and/or a dental restoration such as a crown, bridge or denture. However, any device, such as a dental fixture, intended for implantation may alone be referred to as an implant even if other parts are to be connected thereto. Dental implants are presently preferred embodiments.

The present invention relates to a coated metal implant to be used as medical and/or dental implant, which actively facilitates controlled adhesion of hard and/or mineralized tissue to the implant, e.g. which actively induces adhesion of hard and/or mineralized tissue to the implant and/or exhibits improved effect on bone remodeling and/or biocompatibility of the implant due to at least part of its surface being coated with a low concentration layer of polyunsaturated fatty acids (PUFA). In a particular embodiment, at least part of its surface is coated with a low concentration layer of polyunsaturated fatty acids (PUFA) in combination with a phospholipid, vitamine and/or antioxidant.

The present invention at the same time discloses a metal implant to be used as medical and/or dental implant, which actively inhibits hard and/or mineralized tissue adhesion to the implant, such as bone attachment, due to at least part of its surface being coated with a layer of polyunsaturated fatty acids (PUFA) in a high concentration. It is furthermore understood that the later embodiment of the present invention, i.e. the implant which actively inhibits hard and/or mineralized tissue adhesion to the implant, such as bone attachment, due to at least part of its surface being coated with a layer of polyunsaturated fatty acids (PUFA) in a high concentration, may still induce and/or actively promote bone remodeling in tissue that is not in direct contact with the implant. Thus, in such an embodiment, a slippery implant is generated that is easily removable from the implantation site, which still displays a beneficial effect on mineralized and/or hard tissue growth in the general vicinity of the implantation site. Without a wish to limit the scope of the present invention to a particular theory, the above discussed beneficial effect of a high concentration coating of PUFA of a slippery implant on close by mineralized and/or hard tissue is most likely due to the natural dilution of the PUFA once it is released into the surrounding tissue of the implant, which will of course generate a sufficiently lower concentration of PUFA at an easily calculatable distance from the actual contact surface of the implant.

The invention further discloses a method for manufacturing said metal implant with either inducing or inhibiting effect on hard and/or mineralized tissue adhesion and/or bone remodeling, wherein the implant is coated with PUFA at a specific concentration and irradiated with UV light.

The device or implant according to the invention can be used for a number of purposes. Examples of such purposes include use for: inducing local hard and/or mineralized tissue (e. g. bone tissue) formation at the implantation site; controlling microbial growth and/or invasion at the implantation site or systemically; reducing inflammation at the implantation site or systemically; stimulating ligament repair, regeneration or formation; inducing cartilage formation; nucleating, controlling and/or templating biomineralization; improving attachment between implants and tissues; improving osseointegration of implants; improving tissue adherence to an implant; hindering tissue adherence to an (semi permanent or temporary) implant; improving contact between tissues or tissues and implants, improving tissue sealing of a (surgical) wound; inducing apoptosis (cell death) in unwanted cells (e.g. cancer cells); inducing specific cell differentiation and/or maturation, increasing tissue tensile strength; improving wound healing; speeding up wound healing; templating tissue formation; guiding tissue formation; local gene therapy; stimulating nerve growth; improving vascularisation in tissues adjacent to an implant; stimulating local extracellular matrix synthesis; inhibiting local extracellular matrix breakdown; inducing local growth factor release; increasing local tissue metabolism; improving function of a tissue or body-part; reducing local pain and discomfort. The purpose will depend on the type of implant as well as the nature and/or concentration of the PUFA.

Presently preferred embodiments may improve the osseointegration of implants; i.e. they improve tissue adherence to an implant, improve bone remodeling, hinder tissue adherence to a (semi -permanent or temporary) implant, reduce bone remodeling, and/or improve contact between tissues or tissues and implants. It is presently envisioned that an implant does either display a stimulating or a dampening effect on bone remodeling and/or osseointegration, but it is of course also possible to produce an implant that does display these contrary effects on different parts of the implant.

The term "hard and/or mineralized tissue" is in the present context employed to describe a variety of different naturally occurring tissue types that have become mineralized, and/or tissue having a firm intercellular substance. A hard and/or mineralized tissue according to the present invention is preferably selected from the group consisting of cartilage, bone, dental enamel, dentine-like tissue, dental hard tissue, and cortical tissue.

In general, mineralized tissue is vital to many characteristic adaptive phenotypes in vertebrates. Three primary tissues, enamel (enameloid), dentin, and bone, are found in the body armor of ancient agnathans and mammalian teeth, suggesting that these two organs are homologous. Mammalian enamel forms on enamel-specific proteins such as amelogenin, whereas dentin and bone form on collagen and many acidic proteins, such as SPP1, coordinately regulate their mineralization.

In a presently preferred embodiment, the implant comprises at least 90% of weight of a metal material.

It is presently preferred that the metal material is titanium or an alloy thereof, e.g. an alloy with zirconium, tantalum, hafnium, niobium, aluminum, vanadium, chrome, cobalt, magnesium, iron, gold, silver, copper, mercury, tin or zinc. In a particularly preferred embodiment, the metal material is titanium.

In an embodiment. the metal material of the coated metal implant is selected from the group consisting of titanium, titanium alloy, zirconium, hafnium, tantalum, niobium, and mixtures of two or more of zirconium, hafnium, tantalum and niobium.

Also, preferably the metal material is zirconium, hafnium, tantalum, niobium or mixtures of two or more of these. The metal material preferably also is a metal hydride, such as TiH, metal hydroxide, such as TiOH, a hydride of an alloy, or a hydroxide of an alloy. Alternatively the material may be an oxide of a metal. Also, the implant material may be aluminium, gold or surgical steel nickel.

The term "cp" is well known to the person skilled in the art and stands for "commercially pure" and relates to the level of pureness of the employed metal, such as Ti.

When the metal material is an alloy of titanium, zirconium, tantalum, hafnium or niobium, it may be an alloy between one or more of these metal elements; or it may be an alloy containing one or more other metals such as aluminium, vanadium, chrome, cobalt, magnesium, iron, gold, silver, copper, mercury, tin or zinc; or both.

In a presently preferred embodiment, the implant comprises at least 90% of weight of titanium and/or an alloy of titanium.

In one embodiment of the invention, the Ti comprising implant is exposed to UV radiation prior, simultaneously and/or after coating with a PUFA. $TiO_2$ is a well known photocatalyst (Nakamura et al., 2002). If the surface is irradiated with UV light, electron-hole pairs are generated, reactive oxygen compounds are released, and water molecules dissociate and adsorb at the surface. Those hydroxide groups cause an increased hydrophilicity of the surface. The efficacy of UV irradiation has been shown previously by contact angle (CA) measurements and, to a certain extent, by FTIR measurements (Nakamura et al., 2002; Miyauchi et al., 2002).

In one embodiment, the present invention relates to a method to coat a cp Ti medical implant with a thin layer of PUFA, e.g. as demonstrated in Examples 1 and 2 with eicosapentaenoic acid (EPA), alternatively the implant is additionally exposed to UV irradiation and washing (chemically bound PUFA coating) or only washed (physically adsorbed PUFA coating). In the presently presented examples, the changed surface characteristics of the cp Ti implant are then detected and characterize with physical and chemical methods.

In the present context PUFA stands for polyunsaturated fatty acids, a term well known to the person skilled in the art to include a well defined group of fatty acids.

**Table 1.** Common Fatty Acids

| Chemical Names and Descriptions of some Common Fatty Acids | | | | |
|---|---|---|---|---|
| Common Name | Carbon Atoms | Double Bonds | Scientific Name | Sources |
| Butyric acid | 4 | 0 | butanoic acid | butterfat |
| Caproic Acid | 6 | 0 | hexanoic acid | butterfat |
| Caprylic Acid | 8 | 0 | octanoic acid | coconut oil |
| Capric Acid | 10 | 0 | decanoic acid | coconut oil |
| Lauric Acid | 12 | 0 | dodecanoic acid | coconut oil |
| Myristic Acid | 14 | 0 | tetradecanoic acid | palm kernel oil |
| Palmitic Acid | 16 | 0 | hexadecanoic acid | palm oil |
| Palmitoleic Acid | 16 | 1 | 9-hexadecenoic acid | animal fats |
| Stearic Acid | 18 | 0 | octadecanoic acid | animal fats |
| Oleic Acid | 18 | 1 | 9-octadecenoic acid | olive oil |
| Ricinoleic acid | 18 | 1 | 12-hydroxy-9-octadecenoic acid | castor oil |
| Vaccenic Acid | 18 | 1 | 11-octadecenoic acid | butterfat |
| Linoleic Acid | 18 | 2 | 9,12-octadecadienoic acid (n-6) | grape seed oil |
| Alpha-Linolenic Acid (ALA) | 18 | 3 | 9,12,15-octadecatrienoic acid (n-3) | flaxseed (linseed) oil |
| Gamma-Linolenic Acid (GLA) | 18 | 3 | 6,9,12-octadecatrienoic acid (n-6) | borage oil |
| Arachidic Acid | 20 | 0 | eicosanoic acid | peanut oil, fish oil |
| Gadoleic Acid | 20 | 1 | 9-eicosenoic acid | fish oil |
| Arachidonic Acid (AA) | 20 | 4 | 5,8,11,14-eicosatetraenoic acid (n-6) | liver fats |
| EPA | 20 | 5 | 5,8,11,14,17-eicosapentaenoic acid (n-3) | fish oil |
| Behenic acid | 22 | 0 | docosanoic acid | rapeseed oil |
| Erucic acid | 22 | 1 | 13-docosenoic acid | rapeseed oil |
| DHA | 22 | 6 | 4,7,10,13,16,19-docosahexaenoic acid (n-3) | fish oil |
| Lignoceric acid | 24 | 0 | tetracosanoic acid | small amounts in most fats |
| **Table 1 (continued)** | | | | |

As is well known in the art, a typical PUFA may be described by the following formulae:

$$H_3C\text{-}n_x\text{-}((n_x(=)n_x)_y\text{-}n_x\text{-}((n_x(=)n_x)_y\text{-}n_x\text{-}COOH$$

wherein:

> n is any natural and/or artificially modified C and/or any other natural and/or artificial element that can form a double binding, and
> (=) represents a double binding, and
> x is a number between 0 and x, and
> y is a number between 1 and x, and

wherein the position of the double binding is variable along the chain of n.

The double bounds can be either all in *cis* or all in *trans,* or in mixed configurations.

Polyunsaturated fatty acids have effects on diverse physiological processes impacting normal health and chronic diseases. The predominant sources of PUFA are vegetable oils and fish. Chemically, PUFA belong to the class of simple lipids, as are fatty acids with two or more double bonds in cis position. The location of the first double bond, counted from the methyl end of the fatty acid, is designated by the omega- or n-number.

PUFA occur throughout animal, plant, algae, fungi and bacteria and is found widely in many lipid compounds such as membranes, storage oils, glycolipids, phospholipids, sphingolipids and lipoproteins. PUFA is produced commercially from selected seed plants, and some marine sources.

There are two main families of PUFA, n-3 and n-6 (see Table 1).
Typical PUFA are:

> 3-Series PUFA, n-3: Alpha-linolenic acid (ALA), eicosapentaenoic acid (EPA), docosahexaenoic acid (DHA)
> 6-Series PUFA, n-6: linoleic acid, gamma-linolenic acid (GLA), arachidonic acid (AA)

Preferably, the PUFA is selected from the group consisting of n-3 and n-6 fatty acids.

Dietary PUFA have long been recognized as being able to exert unique influences on metabolic pathways and cellular growth. For example, they can act as hormones and control the activity of transcription factors. What is more, PUFA were found to elicit changes in gene expression that precede changes in membrane composition by directly governing the activity of nuclear transcription factors. The effects exerted by PUFA are most likely mediated via changes in membrane composition, altered hormone release or signalling, and/or ligand/receptor interaction.

In general, fatty acids (FA), the basic modules of lipids have a great influence on properties of the cell membrane (e.g. fluidity) and on cell metabolism. Dietary FA have prior been shown to influence bone modelling and remodelling. Especially the n-6/n-3 ratio of polyunsaturated fatty acids (PUFA) is of implicit importance. EPA (eicosapentaenoic acid 20:5 n-3) is a precursor in the production of eicosanoids in the body, a group of signalling molecules that can as well be synthesized from other n-3 and n-6 PUFA, such as AA (arachidonic acid 20:4 n-6) and DHA (docosahexaenoic acid 22:6 n-3). Those PUFA have opposing effects on, amongst others, bone metabolism and inflammation.

In an embodiment, the PUFA used in the present invention is selected from the group consisting of eicosapentaenoic acid, arachidonic acid, docosahexaenoic acid and mixtures thereof.

Preferably, the PUFA used in the present invention comprises or consists of EPA.

Although in one presently preferred embodiment, EPA was chosen as coating substance for a metal surface, such as a Ti surface of an implant, it is well documented in the field that DHA acts in a similar way with regards to bone tissue stimulation. Thus in another embodiment, DHA or mixtures between EPA and DHA are envisioned as surface coating as well.

Bone is a multifunctional organ that consists of a structural framework of mineralized matrix and contains heterogeneous populations of chondrocytes, osteoblasts, osteocytes, osteoclasts, endothelial cells, monocytes, macrophages, lymphocytes and hemopoietic cells. Bone growth is regulated by complex interactions between different intercellular and extracellular players. Without the intention to be bound by a specific scientific hypothesis, it is envisioned that PUFA in a low concentration have a beneficial effect on bone growth, formation, resorption and/or adhesion, whereas a high concentration of PUFA is repellent to and actively inhibits bone growth, formation and/or adhesion.

Thus, the present invention relates to a metal implant to be used as medical and/or dental implant, which actively facilitates controlled adhesion of bone to the implant, e.g. which actively induces adhesion of bone to the implant and/or exhibits improved effect on bone remodelling and/or biocompatibility of the implant due to at least part of its surface being coated with a low concentration layer of polyunsaturated fatty acids (PUFA) and irradiated with UV light, before and/or after the coating. The present invention at the same time discloses a metal implant to be used as medical and/or dental implant, which actively inhibits bone adhesion to the implant, such as bone attachment, due to at least part of its surface being coated with a layer of polyunsaturated fatty acids (PUFA) in a high concentration. The invention further discloses a method for manufacturing said metal implant with either inducing or inhibiting effect on bone adhesion and/or remodelling, wherein the implant is coated with PUFA at a specific concentration and irradiated with UV light.

The invention also discloses to a method for manufacturing a metal implant with an improved effect on adhesion of mineralized and/or hard tissue to the implant, comprising

a) treating the implant with a solution comprising PUFA; and
b) irradiating at least part of the surface of the implant with UV light for at least 30 seconds.

Also, the invention discloses a method for manufacturing a metal implant with an improved effect on adhesion of mineralized and/or hard tissue to the implant, comprising

a) irradiating at least part of the surface of the implant with UV light for at least 30 seconds; and
b) treating the implant with a solution comprising PUFA.

The invention also discloses a method for manufacturing a metal implant with an improved effect on adhesion of mineralized and/or hard tissue to the implant, comprising

a) mirror polishing and/or grit-blasting the implant,
b) washing,
c) autoclaving,
d) treating the implant with a solution comprising PUFA; and
e) irradiating at least part of the surface of the implant with UV light for at least 30 seconds.

Further, the invention discloses a method for manufacturing a metal implant with an improved effect on adhesion of mineralized and/or hard tissue to the implant, comprising

a) mirror polishing and/or grit-blasting the implant,
b) washing,
c) autoclaving,
d) irradiating at least part of the surface of the implant with UV light for at least 30 seconds; and
e) treating the implant with a solution comprising PUFA.

In the methods for manufacture of an implant of the invention, only part of the implant may be treated with a solution comprising PUFA.

Preferably the at least part of the surface of the implant irradiated with UV light is the part treated with a solution comprising PUFA.

In the methods of the invention for manufacturing a metal implant with an improved effect on adhesion of mineralized and/or hard tissue to the implant the solution comprising PUFA preferably comprises EPA.

Preferably, in the methods for manufacturing a metal implant with an improved effect on adhesion of mineralized and/or hard tissue to the implant, the implant preferably comprises titanium.

In a presently preferred embodiment, the invention thus discloses a method for manufacturing a metal implant with an improved effect on adhesion of mineralized and/or hard tissue to the implant, wherein the surface is irradiated with UV light characterized by Fluo.link, A = 312 nm.

In an equally preferred embodiment, the invention further discloses a method for manufacturing a metal implant with an improved effect on adhesion of mineralized and/or hard tissue to the implant, wherein intensity of the UV light which the surface is irradiated with is approximately 6 mW/cm$^2$.

*In vitro* experiments documented in the experimental section (see Example 1) were carried out to test cytotoxicity and cell attachment of MC3T3-E1 cells to EPA-modified titanium implants. The surface characterization of the implants described herein show that the surface structure of titanium was not changed during the procedure, while chemical properties of the titanium surfaces and of FA were changed in order to form chemical bindings. Significant amounts of $^{14}$C-EPA were found to be left on surfaces of UV-irradiated implants compared to non-irradiated implants after thorough washing with methanol. *In vitro* results on MC3T3-E1 cells showed that a high amount of EPA was toxic to the cells. However, surfaces with lower amounts of EPA bound to the surface with UV light showed lower toxicity and higher cell attachment compared to untreated implants and uncoated implants.

In conclusion, the present results demonstrate that UV light is a suitable method to bind PUFA, e.g. EPA, and to create a thin layer of PUFA on the surface of e.g. titanium implants, which has shown to improve the cell viability and attachment of osteoblastic cells.

UV light in the range of UV C (100 nm - 280 nm) and/or UV B light (280 nm - 315 nm) is preferred for the present invention. For example, 254 nm and 302 nm have been proven to work well for the present invention. The activity of titanium is reduced from about 350 nm to 400 nm. Consequently UV B is a safe area for $TiO_2$ activation (S.-M. Oh et al. 2003).

EPA is susceptible to light-induced oxidation due to unsaturation in the structure. Especially the highly energetic UV light leads to oxidation and generation of reactive oxide groups. As is shown in Example 2, analysis of FTIR spectra showed that changes occurred in the EPA structure. Measurements of the amount of $^{14}$C-labelled EPA on surfaces showed that a measurable amount of $^{14}$C-EPA was left on the non-irradiated and irradiated surfaces. However, the significant difference in measured $^{14}$C signals indicated that the irradiated surface coating was stable against thorough washing and therewith chemically bound. The fatty acid film on irradiated surfaces may consist of a combination of chemically and physically bound EPA. Surface roughness of the polished cp Ti samples precluded detailed analysis of the coating structure with for example grazing incidence x-ray reflectometry, where a $S_a$ not higher than 1 nm is required; such a low surface roughness could e.g. be obtained by sputtering a thin layer of Ti onto smooth Si surfaces.

In Example 2, the 2 reference groups with physically adsorbed EPA on their surfaces showed that large quantities of EPA (group A) had a toxic effect on the cells, and small quantities of EPA (group B) had no significant effect on cell attachment. For group C it was found that UV-irradiation of $TiO_2$ alone (control in group C) had a slightly negative effect on cell attachment while toxicity was not increased.

Chemically bound EPA in group C had a positive effect on cell attachment and was shown to be nontoxic. Therefore it can be suggested that EPA is effective even though it is chemically bound to the surfaces and therefore not directly available for the cells. A surface coating with EPA may change the characteristics of the protein layer readily adsorbed onto the surfaces of the samples when in contact with protein-containing media. A possible explanation for this phenomenon is that EPA is prone to autoxidation on air due to its unsaturation; thus the results of the in vitro tests, which showed a positive effect of the coating by irradiation.

Furthermore, studies are included which investigate Ti surfaces, which alone are activated with UV-irradiation and such surfaces to which the EPA is given to the surfaces subsequently. By this specific measure, the alteration of the fatty acid molecules may be reduced.

Implant surfaces are exposed to high friction forces during implantation; thus a surface coating should be thin and firm to stand high abrasion forces. Thus the present invention in a presently preferred embodiment relates to implants with a chemical surface coating of PUFA.

As was shown in CA measurement in the experimental section, surface roughness parameters did not change due to surface modification procedure, indicating that all changes of surface contact angles discussed were most likely caused by changes in surface chemistry.

A preferred cleaning solvent, such as methanol, leaves a thin layer of carboneous residues on surfaces. Higher carbon content on metal surfaces has prior been reported to increase hydrophobicity of those surfaces. This explains the increase in CA for the positive control in group B. The comparably lower CA of the surface coated coins in group B indicated that most probably, and in accordance with the amount of $^{14}$C-EPA measured on those surfaces, a thin layer of physically adsorbed EPA was left after washing with methanol. For chemical coating (group C), reactive sites had to be generated both, on the Ti surface and within EPA molecules to create a thin and stable surface layer. The photocatalytic effect of UV light on $TiO_2$ was utilized for generation of reactive hydroxide groups on the surfaces of the Ti coins. As is well known in the art, $TiO_2$ becomes amphiphilic due to UV-irradiation, with a structure containing of coexisting hydrophilic and lipophilic phases. In the presently presented experiments, CA measurements with water showed that irradiated $TiO_2$ surfaces became more hydrophilic.

Analysis with the Profilometer showed that EPA was unevenly distributed on non-irradiated surfaces (group A); drop-like structures, or rather regions with more or less thick EPA layer were observed (data not shown). However, the fatty acids were evenly spread on the surfaces after irradiation of the samples, which was most probably due to lipophilic properties of the surfaces.

Consequently, the present invention in one aspect relates to a coated medical implant, which is at least in part coated with an evenly spread thin layer of PUFA and irradiated with UV light, before and/or after the coating as defined herein. Such an implant is in a presently preferred embodiment coated with chemically bound PUFA, such as with EPA, AA and/or DHA. An implant corresponding to said particular embodiment is further characterized by exhibiting improved effect on hard and/or mineralized tissue adhesion and/or bone formation, remodeling, addition and/or biocompatibility.

In the present context, a thin layer of PUFA represents a monolayer of a PUFA, e.g. EPA, representing about $1-5 \times 10^{15}$ bound unsaturated bindings per $mm^2$ titanium surface, such as approximately $3 \times 10^{15}$ bound unsaturated bindings per $mm^2$ titanium surface.

Typically, a high concentration of PUFA per $mm^2$ metal, e.g. titanium surface represents about 1-5 nanomol PUFA /$mm^2$ titanium surface, such as 3.2 nanomol PUFA / $mm^2$ titanium surface. A high concentration coating thus comprises about 0.1-5 $\mu$g PUFA/ $mm^2$ titanium surface, such as at least 1 $\mu$g PUFA/$mm^2$ titanium surface. Typically said PUFA is EPA.

In turn, a low concentration of PUFA per $mm^2$ titanium surface represents less than 0.5 nanomol PUFA/$mm^2$ titanium surface. A low concentration coating thus comprises about 1-100 nanogram PUFA/$mm^2$ titanium surface, such as at the most 10 nanogram PUFA/ $mm^2$ titanium surface or at the most 1 nanogram/$mm^2$ titanium surface. Typically said PUFA is EPA. In an embodiment, the coated metal implant is coated with PUFA at a concentration comprised from 1 to 10 nanogram/$mm^2$.

A presently preferred embodiment is thus a metal implant for controlled mineralized tissue adhesion, wherein at least part of the surface of the implant is coated with PUFA (polyunsaturated fatty acids) at a concentration of less than 0,01 $\mu$mol, such as less than 0,001 $\mu$mol.

In the experiment data, it is in Example 1 shown a better attachment and cell differentiation *in vitro.*

In contrast, it is shown *in vivo* in Example 3, a decreased bone attachment strength with the implants that were UV irradiated (with and without PUFA). The results indicates that surface treatment with UV light, in presence or absence of EPA, reduces bone attachment and volumetric bone mineral density in the peri-implant bone tissue. None of the presented surface treatments showed increased LDH activity levels as a result of tissue necrosis on the adjacent bone. In addition, gene expression of bone formation markers show higher mRNA levels in UV irradiated (with and without PUFA). It can be concluded from this study that surface functionalization of Ti implants with UV light and EPA is a biocompatible coating to reduce bone bonding ability of Ti and maintain new bone formation.

The goal of Example 3 was to produce Ti surfaces with a layer of chemically bound EPA using UV irradiation that maintain the healing process of cortical bone while having a low attachment, to be applicable as bone fracture plates. These applications require the development of surfaces that prevent soft tissue attachment and irritation, allow tissue gliding, but maintain their biocompatible properties. Pure titanium metal is one of the most widely chosen materials for bone plates, because of its excellent biocompatibility and corrosion resistance. The only available strategy today to minimise bony integration is using surfaces with minimal roughness by polishing titanium fixation plates. Although surface roughness has been considered the major determinant in osseointegration, surface chemistry plays an important role as well. Coating Ti with a uniform layer of EPA using UV irradiation provides an example to investigate how surface chemistry modification interacts in their effect on cells and influence osseointegration.

Furthermore, another preferred embodiment is a metal implant coated with PUFA and further characterized in that at least the part of the implant coated with PUFA has been exposed to UV radiation for at least 30 seconds, such as for at least 10 minutes, or for at least 30 minutes. The UV treatment may take place before, after or simultaneously the coating of the implant with PUFA.

In one embodiment, the implant's functionality is further improved by binding and/or integrating one or more various active elements and/or substances to the surface of the implant, either before, simultaneously, and/or after the PUFA coating and alternative UV treatment of the implant. Such bindings are preferably carried out using for example chemical reactants having two reactive functionalities such as formalin or glutaraldehyde. Typically, an element or substance to be bound to the surface of the implant is selected from the group consisting of vitamins (such as vitamin E, C, D, A), in particular fat-soluble vitamins, antioxidants, ions (such as fluoride, calcium, phosphates, carbonates), and antibiotics (such as fat soluble antibiotics, microclines, tetracycline).

In an embodiment, the coated metal implant of the invention additionally comprises fat-soluble vitamins, such as vitamin E.

Vitamin E is preferably present on the implant or device during UV irradiation as it prevents oxidation of PUFA (as demonstrated in Example 4).

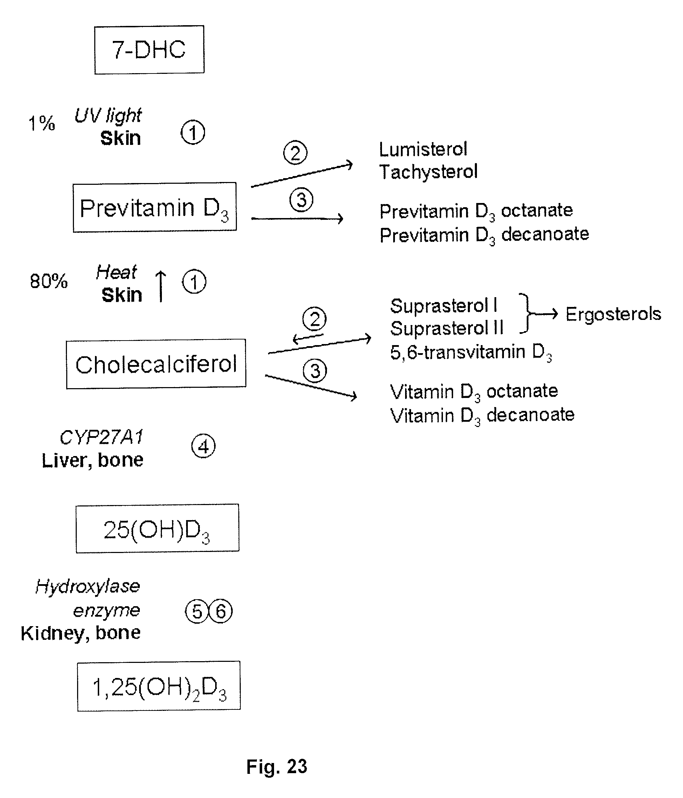

7-Dehydrocholesterol (7-DHC, provitamin $D_3$) is converted to cholecalciferol (vitamin D3) via previtamin $D_3$ in the human skin. Further conversion to $25(OH)D_3$ is mainly reported to happen in the liver. However, since the hydroxylase enzyme CYP27A1 is ubiquitously expressed in other tissues, the system for local production may be also available in bone (Aiba I et al., 2006). Besides the kidneys, $1\alpha,25(OH)2D3$ has as well been shown to be synthesised locally in bone from $25(OH)D_3$ (Ichikawa et al., 1995; Atkins et al., 2007). A schematic overview of the conversion can be found as Figure 23. $1\alpha,25(OH)_2D_3$ is the biological active form of vitamin D3 and acts as a hormone to regulate serum calcium and phosphate levels and is an important factor during bone growth and mineralisation (St-Arnaud R, 2008).

Vitamin-D deficiency has been considered as systemic risk factor for reduced bone mineral density, osteoporosis, and impaired osseointegration. Ageing results in a number of changes in bone and calcium metabolism which can potentially affect osseointegration. The secretion of parathyroid hormone increases with age. Conversely, there is a decrease in calcitonin and Vitamin D absorption with age. The main problem with vitamin D defficiency is that is very difficult to calculate from dietary intake and sun-exposure what the blood level will be.

Therefore, coating 7-DHC to titanium implants and activating the conversion from 7-DHC to cholecalciferol with UV light, with or without PUFA, may affect bone healing positively and improve peri-implant bone healing in normal and osteoporotic patients. Cholecalciferol will be then converted to calcidiol and calcitriol in the bone with their own hydroxylases when needed. 7-DHC has the advantage of having a much lower toxicity compared to cholecalciferol and hydroxilated forms of vitamin D. Besides, irradiation of 7-DHC gives the right end product that can be modified further in the body, while calcitriol might oxidize to other compounds on the titanium surfaces.

Consequently, the present invention also relates to a metal implant wherein the implant additionally comprises 7-dehydrocholesterol. The invention also discloses an implant coated with 7-dehydrocholesterol and subsequently irradiated with UV light to form cholecalciferol (vitamin $D_3$). Such an implant may or may not also have PUFA on its surface. Titanium (Ti) surfaces were coated with either 7-DHC (with or without PUFA in the form of EPA) or cholecalciferol to analyse their transition initiated by UV light in Example 5.

For 7-DHC conversion, the most effective irradiation has wavelengths from 270 to 300 nm although the wave length range of UV C (100 nm - 280 nm) and UV B (280 nm - 315 nm) also are preferred.

Typically, the device or implant specimen is produced aseptically and/or sterile, allowed to air-dry and is then packaged in a sterile, airtight plastic bag in which it is stored until use for implantation. However, sometimes a wet storage system might be desired, e.g. like canning or storage in a fluid like saline or simply an electrolyte from the manufacturing process. Although the coating can be run under aseptic or even sterile conditions, the need for doing this may be avoided by including a sterilization step prior to use, using conventional methods such as ionizing radiation, heating, autoclaving, or ethylene oxide gas etc. The choice of method will depend on the specific characteristics and properties of the implant.

Prior to the coating treatment, the implant should be thoroughly cleaned. This may typically consist of the implant being mechanically pre-treated by electropolishing or sandblasting to modify surface structure if desired, and subsequently thoroughly cleaned using hot caustic soda followed by a de-greasing step, e.g. in concentrated tri-chloroethylene, ethanol or methanol, before being treated in a pickling solution, e.g. hydrofluoric acid, to remove undesired oxides and impurities on the surface. After pickling, the implant specimen is washed thoroughly in hot, double distilled ion-exchanged water.

Although the present invention is mainly concerned with the coating of metal implants with PUFA at specific high or low concentrations, it is equally envisioned that for example a variety of naturally occurring lipids characterized by comprising multiple double bindings can be used in a similar way. In this context, a thin layer of naturally occurring lipids would be considered to represent a monolayer of a naturally occurring lipid, representing about $1\text{-}5\text{x}10^{15}$ bound unsaturated bindings per $mm^2$ titanium surface, such as $3\text{x}10^{15}$ bound unsaturated bindings per $mm^2$ titanium surface.

In an embodiment, when the implant comprises at least 90% of weight of titanium and/or an alloy of titanium, the PUFA comprises or is EPA (eicosapentaenoic acid), then the coating comprises from 1 to $5\text{x}10^{15}$ bound unsaturated bindings of EPA per $mm^2$ titanium surface.

The invention is further illustrated by the following, non-limiting examples.

## *Experimental Section*

### Example 1

Rough surface implant

**Matrix:**

| Type of coin | Concentration of EPA | Treatment | № of coins |
|---|---|---|---|
| Polished | 0 mM<br>10 mM<br>50 mM | irradiated with UV light no irradiation | in vitro 6·5 = 30<br>char. 6·3 = 18 |
| Grit-blasted | 0 mM<br>10 mM<br>50 mM | irradiated with UV light no irradiation | in vitro 6·5 = 30<br>char. 6·3 = 18 |
| | | Total number of coins | 96 |

Cleaning

1. Coins rinsed with pure water
2. Coins washed with ethanol
3. Ultrasonication of coins in water for 5 min

4. Again coins rinsed with pure water

5. Coins allowed to dry on sterile bench

6. Autoclaving of coins

Surface modification

1. Work in sterile bench

2. EPA solution is filtered (0,2 $\mu$m pore size) to sterilise it

3. 10 $\mu$l of respective solution of EPA in methanol is given on the surface of the coins

4. Coin is allowed to dry on sterile bench

5. Part of the coins are irradiated with UV light after the solvent evaporated → Fluo.link, $\lambda$ = 312 nm

→ Time of irradiation: 30 min (first test showed that the contact angle is increasing up to 10 min irradiation and decreases subsequently)

→ Intensity of irradiation ca 6 mW/cm$^2$

6. Wash UV irradiated coins with methanol to remove unbound fatty acids Characterization

1. Profilometer: 150x, 3 coins, 3 places on each coin, measurement area of 200x160$\mu$m$^2$ (i.e. square of 3x3), important are the picture from pl$\mu$ and the surface roughness parameter calculated with SensoMap

2. Contact angle: Drop of 3 $\mu$l on coin, CA is measured, the drop is enlarged 2x with 1 $\mu$l of pure water, after each enlargement the surface CA is measured, the CA is not measured after drying the coin as this could change the structure of the fatty acids on the surface of the coin and therewith the CA

3. FT IR (DRIFT) to characterize the fatty acids on the surface, difference before and after UV irradiation.

**Example 2**

**Smooth surface implant**

The aim of this study was to investigate the effect of EPA coating on Ti surface on osteoblast cell response *in vitro*. Methods to either physically adsorb or chemically bind the layer with EPA were compared, the latter one being produced by UV irradiation of TiO$_2$ surfaces and EPA. Those surface coatings were detected and characterized by physical and chemical analytical methods. *In vitro* experiments were carried out to test cytotoxicity and ability of EPA-modified Ti surfaces to promote cell attachment and osteogenic differentiation of MC3T3-E1 cells.

**Materials and Methods**

**Chemicals**

EPA was purchased from Sigma (St. Louis, MO, USA), radioactive labelled [14C]-EPA was obtained from American Radiolabeled Chemicals, Inc. (St. Louis, MO, USA).

**Titanium implants and treatments**

Cp Ti implants with a diameter of 6.25 mm and a height of 2 mm were machined from cp Ti rods and subsequently grinded and polished (Phoenix 4000, Buehler GmbH, Düsseldorf, Germany) in seven sequences. A special holder was used to polish 96 implants at the time to avoid batch's differences in future surface characterisation. The silicon carbide papers, the porous neoprene for final polishing and the abrasive colloidal silica suspension (OP-S) were supplied by Struers GmbH (Willich, Germany). The first step consisted in grinding all the implants with P500 in water until they were levelled, with 65 N of pressure and a contra-rotation at 250 rpm. The grinding time was then set down to 10 min and the grain size decreased with papers: P800; P1200 and P2500. For the P4000 polishing paper, the OP-S polishing suspension was used in addition. For the last step, a special porous neoprene (MD-Chem) was used at 200 rpm and 50 N of pressure for 9 min in co-rotation with OP-S suspension, and 1 min in co-rotation with water.

After polishing, titanium coins were cleaned before sterilization. They were washed together in a glass beaker with deionised water for 30 s, with 70% ethanol for 30 s, and in ultrasonic bath at 40°C for 5 min in deionised water. The implants were subsequently placed in 40% NaOH solution in a water bath of 40°C for 10 min, sonicated in deionised water for 5 min, and then washed with deionised water until the pH reached 6. Afterwards the implants were sonicated in deionised water at 50°C for 5 min, placed in 50% HNO$_3$ solution at 50°C for 10 min, and sonicated in deionised water

**EP 2 310 059 B1**

for another 5 min. The implants were washed with deionised water until pH reached 6 and were stored in 70% ethanol until use. Before use, the coins were rinsed with water, rinsed with ethanol, sonicated for 5 min at room temperature and rinsed with deionised water. The titanium coins were then sterilised by autoclaving at 121 °C for 15 min.

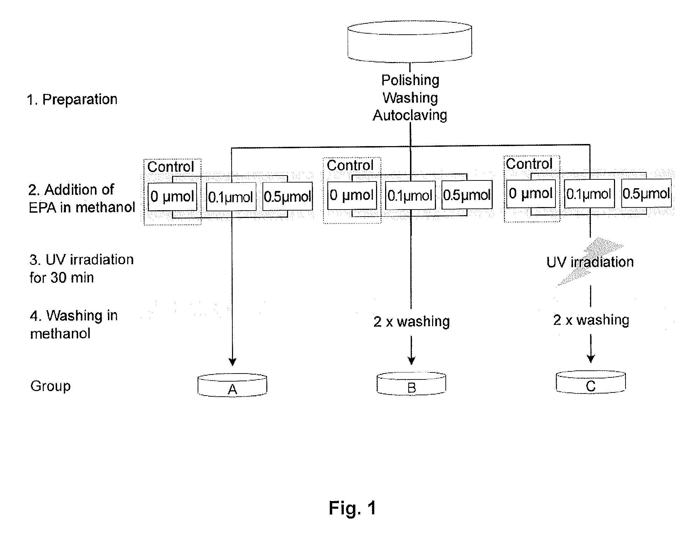

For the surface modification of titanium implants, EPA was dissolved in methanol to 10 mM and 50 mM solution. The EPA solutions were filtered with a 0.22 μm pore size filter before use. Three groups of coins were prepared; non-irradiated and unwashed coins (group A), non-irradiated and washed coins (group B), and UV-irradiated and washed coins (group C). An overview for the 3 different groups is given in Table 2 and Figure 1.

**Table 2: Modification of the different groups of coins as used for in vitro experiments.**

| Group | Modification | Washing | Amount of EPA |
|---|---|---|---|
| A | non-irradiated unwashed | - | 0 μmol<br>0.1 μmol<br>0.5 μmol |
| B | non-irradiated washed | 2 x 1 ml methanol | 0 μmol<br>0.1 μmol<br>0.5 μmol |
| C | irradiated washed | 2 x 1 ml methanol | 0 μmol<br>0.1 μmol<br>0.5 μmol |

Those 3 groups were chosen to show the effects of a thick layer of non-irradiated and therewith physically adsorbed EPA (A), to show the effect of a thin layer of non-irradiated and therewith physically bond EPA (B), and the effect of a thin layer of irradiated and therewith chemically bond EPA (C). For each group a cp Ti coin without any EPA surface coating served as a reference. If EPA was given to the surface, 10 μl of the respective EPA solution was used, which equals an amount of 3.2 nmol EPA/mm$^2$ or 0.99 μg EPA/mm$^2$ for the 10 mM solution and 16.3 nmol EPA/mm$^2$ or 4.93 μg EPA/mm$^2$ for the 50 mM solution. The coins were allowed to dry on air for 15 min in a sterile flow bench. Coins of group C were irradiated with UV light for 30 min (Fluo.link, $\lambda$ = 312 nm, I = 6 mW/cm$^2$). Coins of group B and C were washed with methanol to remove unbound EPA. Group B was washed after drying and group C after irradiation for 30 min. For washing, 1 ml of methanol was given into a microcentrifuge tube, the coin was added and vortexed for 10 s. This was repeated once with fresh methanol. The coins were allowed to air-dry for 15 min in the sterile flow bench and were used immediately for further analysis or cell cultivation.

**Profilometry**

The surfaces of the coins were visualized using the blue light profilometer PLμ 2300 (Sensofar, Terrassa, Spain). Pictures were taken at 50 x magnification and the final picture consists of 3 x 3 assembled pictures (663 x 497 μm$^2$). Three non-overlapping pictures were taken on each coin at random positions.

**Contact angle measurements**

The contact angles (CA) of the surfaces of the different titanium implants were analyzed using a video-based contact angle system (OCA 20, DataPhysics Instruments GmbH, Filderstadt, Germany). The fluid used was deionised water. A drop of 6 μl was given to the surface with a velocity of 0.6 μl s$^{-1}$. The drop shape was recorded with a camera (4.17 pictures s$^{-1}$) and contact angles were measured afterwards from the recorded pictures. The measurements took place at 22 °C. Three samples were analysed for each modification group. One drop was placed on each sample.

**Diffuse reflectance Infrared Fourier Transform (DRIFT) spectroscopy**

Thick films of lipids and the changes caused by UV irradiation were analysed with FTIR Spectrum 100, Perkin Elmer (USA) The diffuse reflectance unit (DRIFT) was used to collect the spectra. A cleaned, sterilised, but unmodified polished Ti coin served as reference. 10 μl of 50 mM EPA solution was given to the surface of a polished Ti coin. The FTIR spectra were collected before and after irradiation with UV light at different time points of irradiation. The spectra were collected from 4000 cm$^{-1}$ to 450 cm$^{-1}$ with a resolution of 2 cm$^{-1}$. Each spectrum was the result of 8 single spectra. The spectra were baseline corrected with the program Spectrum (Version 6.1.0) from PerkinElmer.

**Amount of EPA on implant surfaces**

In order to determine the amount of $^{14}$C labelled EPA on the surfaces of polished coins in the different groups, their surfaces were modified with 10 $\mu$l of 10 mM EPA solution which contained 1% [$^{14}$C]-EPA. The coins were either non-irradiated or irradiated for 30 min with UV light before washing. Three coins were produced for each group. Each coin was transferred to 5 ml of Insta-gel II Plus liquid scintillation fluid (Packard, Groningen, Netherlands), incubated over night and then measured for 3 min in a liquid scintillation counter (1500 Tri-Carb Liquid scintillation Analyzer, Packard Instrument Co., USA).

**Cell cultures**

The murine osteoblast cell line MC3T3-E1 (DSMZ, Braunschweig, Germany) was used as in vitro model. Cells were routinely cultured at 37°C in a humidified atmosphere of 5% $CO_2$ and maintained in alpha-MEM (PAA Laboratories GmbH, Austria) supplemented with 10% fetal calf serum (PAA Laboratories GmbH, Austria) and 50 IU penicillin/ml and 50 $\mu$g streptomycin/ml (Sigma, St. Louis, MO, USA). Cells were subcultured 1:4 before reaching confluence using PBS (PAA Laboratories GmbH, Austria) and trypsin/EDTA (Sigma, St. Louis, MO, USA). To test the different surface modification of titanium implants with EPA, coins were placed in a 96-well plate and $10^4$ cells were seeded on each coin. The same number of cells was cultured in parallel in plastic in all the experiments.

**Cell viability study**

Lactate dehydrogenase (LDH) activity was used as an index of cytotoxicity in the culture media. After 24 h, the culture media was collected, centrifuged at 500 x g for 5 min at 4°C. The supernatant was stored at 4°C. LDH activity was determined spectrophotometrically according to the manufacturer's kit instructions (Cytotoxicity Detection kit, Roche Diagnostics, Mannheim, Germany), and presented relative to the LDH activity in the medium of cells seeded on plastic (low control, 0% of cell death) and on plastic adding 1% Triton X-100 (high control, 100% cell death), after subtracting the absorbance value obtained in the culture medium alone without cells (background control), using the equation:

$$\text{Cytotoxicity (\%)} = (\text{exp. value} - \text{low control})/ (\text{high control} - \text{low control}) \times 100$$

**Cell attachment study**

The number of cells attached to each coin was measured using DNeasy Tissue Kit (Qiagen Inc, Valencia, CA, USA), which uses silica-gel-membrane technology for rapid and efficient purification of total cellular DNA without organic extraction or ethanol precipitation. The buffer system is optimized to allow direct cell lysis followed by selective binding of DNA to the DNeasy membrane. The number of cells attached to the implants was calculated after measuring DNA concentration at 260 nm using a Nanodrop spectrophotometer (NanoDrop Technologies, Wilmington, DE, USA) and taking into account that the DNA content in $10^6$ mouse cells is about 5.8 $\mu$g.

**RNA isolation and Real-time RT-PCR**

Total RNA was isolated using a monophasic solution of phenol and guanidine isothiocyanate (Trizol, Invitrogen Life Technologies, Carlsbad, CA, USA), following the instructions of the manufacturer. RNA was quantified at 260 nm using a Nanodrop spectrophotometer (NanoDrop Technologies, Wilmington, DE, USA).

The same amount of total RNA (0.5 $\mu$g) from each sample was reverse transcribed to cDNA at 42 °C for 60 min in a final volume of 40 $\mu$l, using iScript cDNA Synthesis kit (BioRad Laboratories, Hercules, CA, USA) that contains both oligo(dT) and random hexamers. Each cDNA was diluted 1/5 and aliquots were frozen (-20°C) until the PCR reactions were carried out. Real-time PCR was performed for two housekeeping genes: 18S ribosomal RNA (18S rRNA), glyceraldehyde-3-phosphate dehydrogenase (GAPDH), and five target genes: osterix (Osx), alkaline phosphatase (ALP), collagen type I (coll-I), osteocalcin (OC), and bone sialoprotein (BSP). Sequences of sense and antisense primers were as follows: 5'-GTAACCCGTTGAACCCCATT-3' (SEQ ID NO:1) and 5'-CCATCCAATCGGTAGTAGCG-3' (SEQ ID NO:2) for 18S rRNA; 5'-ACCCAGAAGACTGTGGATGG-3' (SEQ ID NO:3) and 5'-CACATTGGGGGTAGGAACAC -3' (SEQ ID NO:4) for GAPDH; 5'-AGAGCATGACCGATGGATTC-3' (SEQ ID NO:5) and 5'-CCTTCTTGAGGTTGCCAGTC-3' (SEQ ID NO:6) for coll-I; 5'-ACTGGCTAGGTGGTGGTCAG-3' (SEQ ID NO:7) and 5'-GGTAGGGAGCTGGGTTAAGG -3' (SEQ ID NO:8) for Osx; 5'-AACCCAGACACAAGCATTCC -3' (SEQ ID NO:9) and 5'-GAGAGCGAAGGGTCAGTCAG-3' (SEQ ID NO:10) for ALP; 5'-CCGGGAGCAGTGTGAGC-TTA-3' (SEQ ID NO:11) and 5'-TAGATGCGTTTGTAGGCGGTC-3' (SEQ ID NO:12) for OC; 5'-GAAAATGGAGACGGCGATAG -3' (SEQ ID NO:13) and 5'-ACCCGAGAGTGTGGAAAGTG -3' (SEQ ID NO:14) for BSP.

Real-time PCR was performed in the iCycler (BioRad Laboratories, Hercules, CA, USA) using SYBR green detection. Each reaction contained 5 $\mu$l of cDNA, 500 nM of the sense and antisense specific primers (for all, except for collagen-I which was 300 nM), 12.5 $\mu$l of 2X iQ SYBR Green Supermix in a final volume of 25 $\mu$l. The amplification program consisted of a preincubation step for denaturation of the template cDNA (3 min 95 °C), followed by 40 cycles consisting of a denaturation step (15s 95°C), an annealing step (15s 60°C; for all, except for ALP which was 65°C) and an extension step (30s 72°C). After each cycle, fluorescence was measured at 72°C. A negative control without cDNA template was run in each assay. Samples were run in duplicate.

Real-time efficiencies were calculated from the given slopes in the iCycler software using serial dilutions, showing all the investigated transcripts high real-time PCR efficiency rates, and high linearity (r>0.99) when different concentrations were used. PCR products were subjected to a melting curve analysis on the iCycler and subsequently 2% agarose/TAE gel electrophoresis to confirm amplification specificity, $T_m$ and amplicon size, respectively.

**Statistics**

All data are presented as mean values $\pm$ SEM. Differences between groups were assessed by Students t-test using the program SPSS® for Windows, version 14.0. Results were considered statistically significant at p<0.05 (indicated as *), and p<0.01 (indicated as **).

**Results**

**Effect of UV irradiation on surface hydrophobicity**

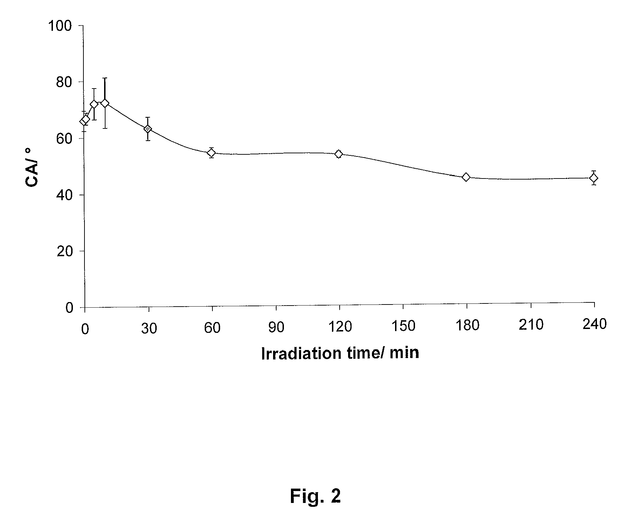

Effect of UV irradiation on CA of $TiO_2$ surfaces was examined to gain information about the irradiation time that should be applied in later experiments. Water CA was measured before irradiation and after an irradiation time of 1, 5, 10, 30, 60, 120, 180, and 240 min. The average CA for the 3 coins at each time point is given in Figure 2. The CA was increased during early irradiation and decreasing subsequently. After 30 min of irradiation the CA was lower than the initial CA for all the coins measured. Therefore, an irradiation time of 30 min was used in the subsequent experiments.

**Effect of EPA surface modification on roughness and hydrophobicity**

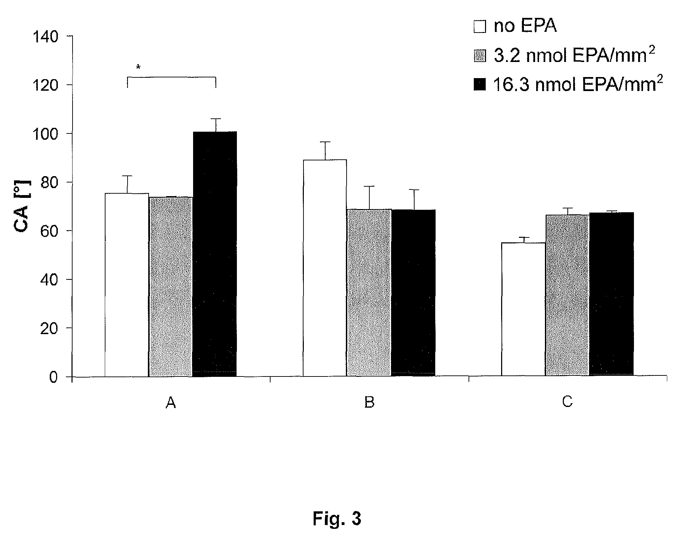

Changes in surface hydrophobicity caused by surface modification were detected by CA measurements. CA values for the 3 groups are given in Figure 3.

The water CA of cleaned, autoclaved Ti surfaces (control A) was 75.5° $\pm$ 7.0°. If 3.2 nmol EPA/mm$^2$ was added to those surfaces no significant changes in CA were found for non-irradiated surfaces (73.8° $\pm$ 0.3°); however, a significantly higher CA of 100.5° $\pm$ 5.5° was measured for surfaces with 16.3 nmol EPA/mm$^2$ added.

The surfaces without EPA in group B (control B) had a CA of 88.9° $\pm$ 7.5°, which was higher compared to control A. Ti coins that were coated with EPA and washed with methanol subsequently had a CA of 68.6° $\pm$ 9.3° and 68.4° $\pm$ 8.2° for 3.2 nmol EPA/mm$^2$ and 16.3 nmol EPA/mm$^2$, respectively; a CA that was lower compared to the surface of control B.

As expected from previous experiments, irradiation with UV light for 30 min followed by washing with methanol led to a slight decrease in the surface contact angle to 54.5° $\pm$ 2.4° (control C) compared to control A. Coating with EPA, irradiation for 30 min and washing with methanol led to an increased CA of 65.8° $\pm$ 5.2° for coating with 3.2 nmol EPA/mm$^2$, and 66.8° $\pm$ 1.5° for coating with 16.3 nmol EPA/mm$^2$, compared to control C.

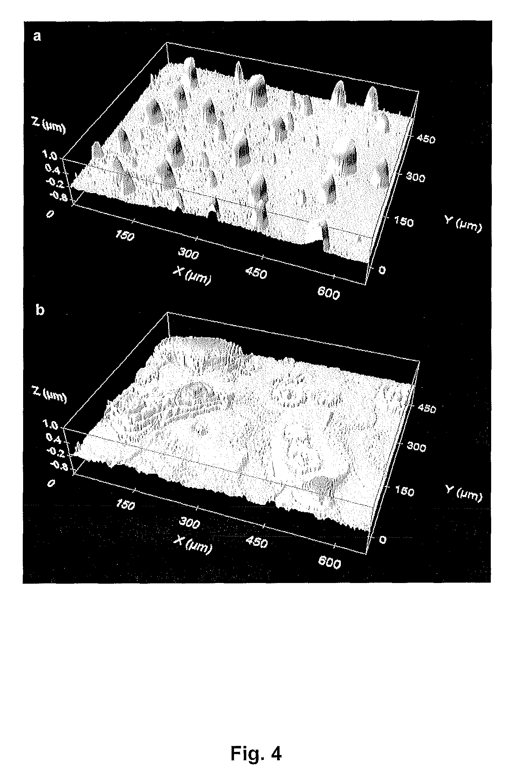

Analysis of the surfaces with Laser Profilometry showed that the surface roughness parameters did neither change by irradiation nor by coating of the surfaces with a thin layer of EPA (i.e. all surfaces of group B and group C, and Control A). Average roughness $S_a$ for those surfaces was 19 nm $\pm$ 2 nm. However, coating with a thick layer of EPA increased $S_a$ to 54.2 nm $\pm$ 13 nm when 3.2 nmol EPA/mm$^2$ was applied to the surface. Profilometric observations of EPA drops show that EPA was unevenly distributed on non-irradiated surfaces (group A) having differently sized drop-like structures (Figure 4a). However, EPA was evenly spread over the whole surface after irradiation with UV light for 30 min (Figure 4b).

**Effect of UV irradiation on the IR-spectrum of EPA**

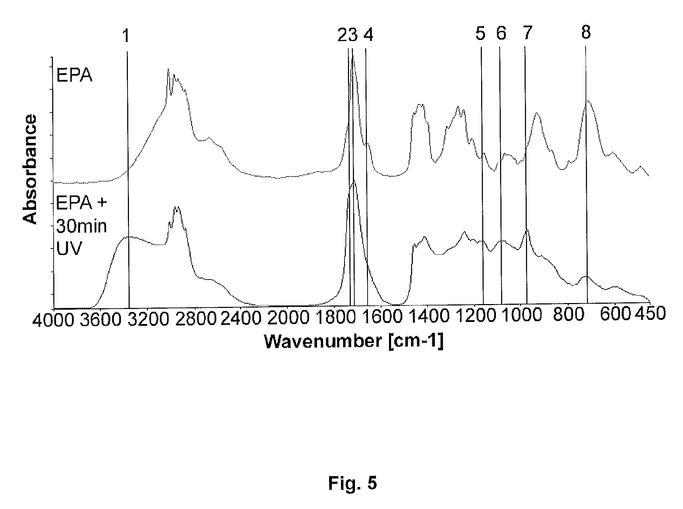

Changes in IR spectra were used to obtain information about how the chemical structure of EPA was changed due to UV irradiation. The absorbance spectra for non-irradiated EPA and EPA which was irradiated with UV light for 30 min are given in Figure 5. Of special interest are the changes in the region -3680-2900 cm$^{-1}$ (1 in Figure 5), the changes of the peaks 1850-1550 cm$^{-1}$ (2-4 in Figure 5) and changes of some absorbances in the fingerprint region (5-8 in Figure 5).

The peak 1 at 3680- 2900 cm$^{-1}$ was not found in the spectrum of non-irradiated EPA but appears in the spectrum of the EPA that was irradiated for 30 min. The peak in the region 1850-1550 cm$^{-1}$ appears to be the result of three absorbances. Two of those three absorbances shifted their position or changed their intensity slightly due to the irradiation. Peak 2 with a maximum at -1730 cm$^{-1}$ was only a shoulder of medium intensity in the non-irradiated sample. However its intensity was increased after irradiation for 30 min with UV light leading to broadening of the apparent peak. Peak 3, with a maximum at -1713 cm$^{-1}$ was very strong both before and after irradiation with UV light. Neither intensity nor position of this absorption changed. Thirdly, a peak of weak to medium intensity with a maximum at -1650 cm$^{-1}$ was found in absorbance spectrum of non-irradiated EPA, in Figure 5 referred to as peak 4. This peak was not visible as a defined peak after irradiation but contributed to a broadening appearance of peak 3.

Many differences in the absorbance spectra of non-irradiated and irradiated EPA were detected in the fingerprint region of the spectra. Most prominent were the increased intensities at 1170 cm$^{-1}$ (peak 5) and 1070 cm$^{-1}$ (peak 6) after UV irradiation, the increased absorbance at -970 cm$^{-1}$ (peak 7), and the decreased absorbance at 710 cm$^{-1}$ (peak 8).

**Amount of EPA on implant surfaces**

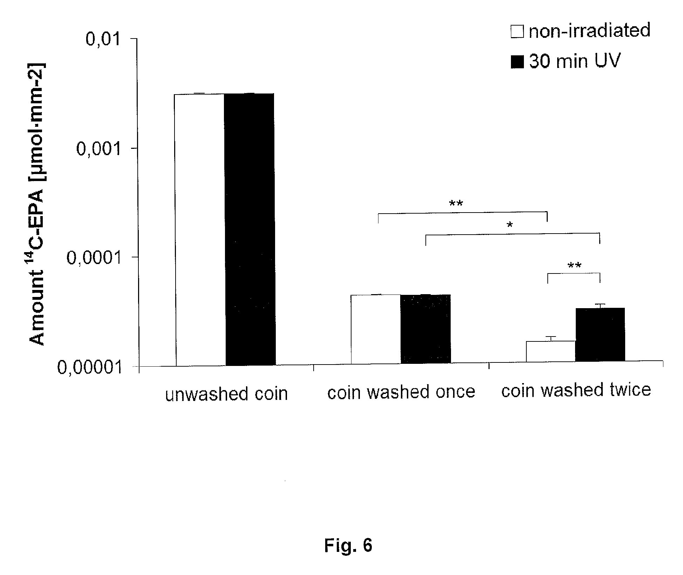

Scintillation counting of $^{14}$C-labelled EPA was used to quantify the amount of EPA on the Ti surfaces and to investigate the efficacy of UV light to chemically bind EPA (Figure 6). The results show that after the first washing in methanol the amount of EPA decreased to the same level for both non-irradiated and irradiated coins. After the second washing in methanol, EPA was still present on non-irradiated and irradiated coins, but there was significantly more EPA on the irradiated surfaces compared to the non-irradiated surfaces.

**Cell attachment to implant surfaces**

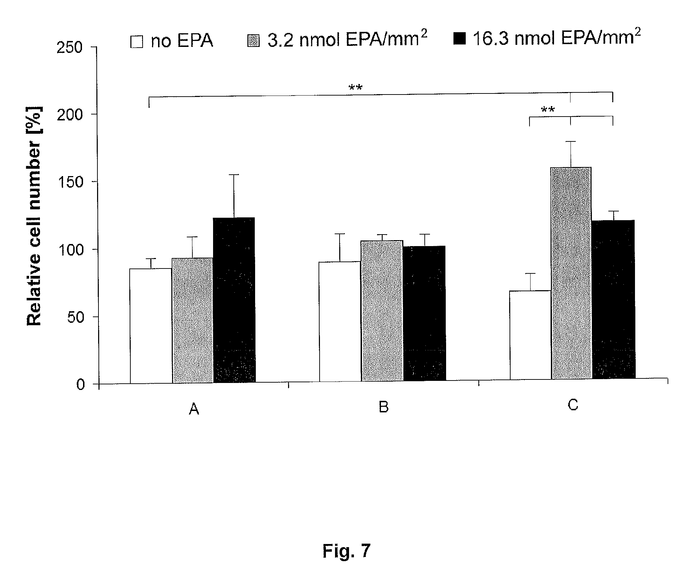

The number of MC3T3-E1 cells attached to the implants was quantified by measuring DNA content (Figure 7). No significant differences in the amount of DNA were detected between the 3 modifications of group A and group B. The relative amount of cells present on surfaces of group C that did not contain EPA was slightly lower compared to pure Ti surfaces of group A. However, the relative amount of cells on the surfaces of group C coated with 3.2 nmol EPA/mm$^2$ and with 16.3 nmol EPA/mm$^2$ was significantly higher compared to the control of group C (p=0.002 and p=0.003, respectively) and also significantly higher when compared to control in group A (p=0.004 and p=0.009 for surfaces coated with 3.2 nmol EPA/mm$^2$ or 16.3 nmol EPA/mm$^2$, respectively). The cell amount was highest on surfaces coated with 3.2 nmol EPA/mm$^2$.

**Effect of EPA surface modification on cell viability**

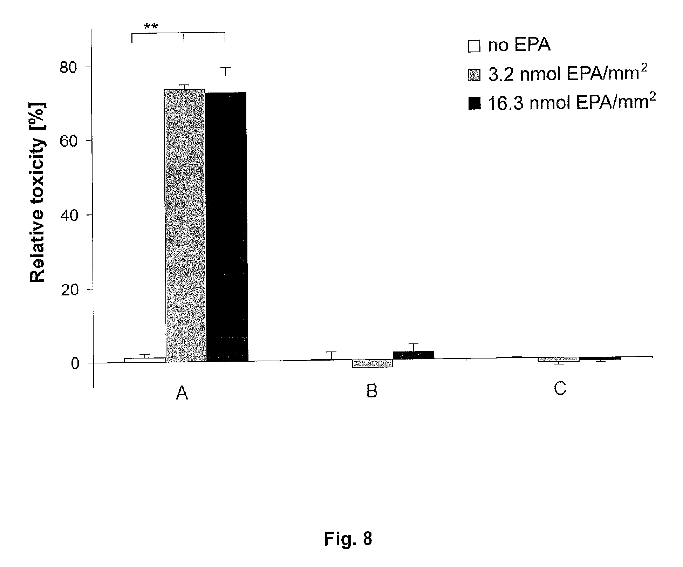

To determine whether different surface modifications with EPA could affect osteoblast cell survival, the LDH activity was measured after 24 h of cultivation (Figure 8). The results showed a significant increase in LDH activity only in group A with implants having a thick layer of EPA on their surfaces, while thin layers of either physically or chemically bound EPA were nontoxic to the cells.

**Discussion**

In this study we compared different methods to coat Ti implant surfaces with EPA by physical adsorption and covalent binding using UV light. We demonstrated that UV light is a suitable method to generate reactive binding sites on TiO$_2$ and within the structure of the EPA molecules. Moreover, in vitro testing showed that implants with UV-bound EPA were able to enhance cell attachment of MC3T3-E1 cells.

Implant surfaces are exposed to high friction forces during implantation; thus to be clinically significant a surface coating should be thin and firm to withstand high abrasion forces. A covalent surface coating could therefore be an interesting option. Such a thin layer of covalently attached molecules may change the surface properties of the material, such as hydrophobicity, pH close to the surface, and structure of the adjacent water layer. Those properties strongly influence the interaction between biomolecules and the surfaces shortly after implantation and may thus facilitate osteoblast attachment and differentiation, which would improve the performance of implants.

TiO$_2$ surfaces became amphiphilic by irradiation with UV light for 30 min, which agreed with previous findings 29. CA measurements with water showed an increased hydrophilicity after an initial decrease. The decrease in hydrophilicity to after 15 min of irradiation with UV light has not yet been described in literature and could be explained by contamination of the surfaces with carbon, an increase in the thickness of the oxygen layer, or slight changes in surface roughness untraceable with profilometry. Profilometric pictures showed that drops of EPA spread on the TiO$_2$ surfaces

after UV irradiation for 30 min indicating increased lipophilicity of $TiO_2$ surfaces. Those results showed that 30 min was an appropriate time for surface irradiation with UV light as the EPA, which was unevenly distributed on the $TiO_2$ surfaces before irradiation, was covering the whole sample surface after 30 min of UV-irradiation.

Surface CA is dependent on chemistry and roughness of surfaces. As average surface roughness $S_a$ did not change due to UV irradiation, coating of the surfaces with EPA and subsequent washing with methanol (such as in group B and C), all changes of surface contact angles measured for those groups were most probably caused by changes in surface chemistry. EPA molecules are hydrophobic and thin layers of EPA were thus increasing the water CA of surfaces. Thick layers of EPA (3.2 nmol EPA/mm$^2$ and 16.3 nmol EPA/mm$^2$ in group A) resulted in soft but rougher surfaces with EPA being unevenly distributed. EPA molecules dissolved in water in small quantities, which decreased the surface tension of water used for the measurements and decreased therewith the resulting CA. Cleaning solvents such as methanol leave a thin layer of carboneous residues on surfaces (Brunette et al., 2001). Higher carbon content on metal surfaces was reported to increase hydrophobicity of surfaces (Hirani et al., 2007). This explains the increased CA for the control in group B that was washed with methanol.

The comparably higher hydrophilicity of the surface coated coins in group B in accordance with the amount of $^{14}$C-EPA measured on those surfaces indicated that a thin layer of physically adsorbed EPA was left after washing with methanol. The decrease in hydrophilicity for the EPA-coated surfaces compared to their control and together with the scintillation counting of $^{14}$C-EPA coated surfaces indicated that a thin layer of covalently bound EPA was left on the surfaces after UV irradiation.

Analysis of FTIR spectra showed the changes that occurred in the EPA structure by irradiation with UV light. Absorbance in the 3680- 2900 cm$^{-1}$ region was strongly related to the appearance of hydroxide groups (-OH) as well as to the generation of peroxide (-OO-) and peroxy acid groups (-CO-OOH) (Socrates et al., 2001) caused by UV irradiation. Absorbance at -1730 cm$^{-1}$ was caused by C=O stretching vibration of esters (Socrates et al., 2001). The increase of the ester peak indicated formation of ester groups but as well the appearance of perester groups (CO-O-O-) after irradiation with UV light (Socrates et al., 2001). Absorbance at 1713 cm$^{-1}$ was caused by C=O stretching vibrations of carboxylic acid groups and did not change in intensity or position (Guillén et al., 1997).

Absorbance with an intensity maximum at -1650 cm$^{-1}$ was associated with stretching vibrations of C=C bonds and its changes in the spectra indicated the appearance of trans double bonds caused by UV-irradiation (Socrates et al., 2001). Increased absorbance at 1170 cm$^{-1}$ and 1070 cm$^{-1}$ again showed the generation of ester groups (CO-O-C asymmetric and symmetric stretch, respectively) by UV irradiation (Socrates et al., 2001). Increased absorbance at 970 cm$^{-1}$ together with a decreased absorbance at 710 cm$^{-1}$ indicated the transformation of cis C=C double bonds to trans C=C double bonds caused by UV irradiation (Socrates et al., 2001). All together, reactive groups, i.e. oxides and their radicals, peroxides and their radicals, peroxy acids, and peresters were generated within the EPA structure by irradiation with UV light. EPA is susceptible to auto- and photooxidation due to unsaturations in the structure. Especially the highly energetic UV light leads to oxidation and generation of reactive oxide groups (Guillén et al., 1997, Cheng et al., 2003, Melø et al., 1988). Photooxidation of EPA may change the chemical structure of the molecules in several ways. One possibility is the formation of oxide groups via peroxide radicals and peroxides in position 5, 6, 8, 9, 11, 12, 14, 15, 17, or 18, together with formation of trans double bonds in the respective positions (Chacon et al., 2000). Another way is degradation of EPA via oxide radicals that are formed in the same positions as for the first possibility, just followed by fragmentation into aldehydes, and the subsequent formation of carboxylic acids and dicarboxylic acids (Rontani et al., 1998) or aldehydes such as malondialdehyde, the volatile end product of fatty acid oxidation that was reported to have negative effects on cell metabolism e.g. by reaction proteins in vivo (Refsgaard et al., 2000). EPA was reported to protect the skin of mice in vivo against UV irradiation, and thereby to protect against photocarcinogenesis (Moison et al., 2001). Those reactive groups may have caused reduction of hydroxide groups and generation of radical oxide groups on the $TiO_2$ surfaces. We hypothesise, that part of the EPA peroxides or their decomposition products that were generated by UV irradiation bound covalently to the $TiO_2$ surfaces via ester, perester, peroxide, and ether bonds (Figure 9).

Measurements of the amount of $^{14}$C-labelled EPA on surfaces showed that a measurable amount of $^{14}$C-EPA was left on both, non-irradiated and irradiated surfaces. However, the significant difference in measured $^{14}$C signals indicated that the irradiated surface coating was more stable against thorough washing than the coating on non-irradiated surfaces. The fatty acid film on irradiated surfaces may consist of a combination of chemically and physically bound EPA and EPA photooxidation products. The data did not support any speculation about how the EPA layer is structured or about how exactly the UV-irradiated EPA is attached to the surface. Surface roughness of the polished cp Ti samples precluded detailed analysis of the coating structure with for example grazing incidence x-ray reflectometry, where a $S_a$ of about 1 nm is required; such low surface roughness could be obtained by sputtering a thin layer of Ti onto smooth Si surfaces (Kim et al., 2004).

Measurements of LDH values and amount of DNA on the surfaces showed that large quantities of EPA (3.2 nmol EPA/mm$^2$ and 16.3 nmol EPA/mm$^2$ in group A) had a toxic effect on the cells, while small quantities of physically bound EPA (group B) had no toxic effect but also significant effect on cell attachment. The toxic effect of high doses of EPA on cell viability has been reported in other cell lines and agrees with the results of the present study.

Chemically bound EPA in group C had a positive effect on cell attachment and was shown to be nontoxic. It can therefore be suggested that EPA could have affected cell attachment, even though it was chemically bound to the surfaces and therefore not directly available for the cells. Surfaces coated with EPA may have changed the characteristics of the protein layer readily adsorbed onto the surfaces of the samples when in contact with protein-containing media. Dependence of adsorption of proteins and cells on surface modifications has been examined by other groups as well (Kim et al., 2005, Cooper et al., 2001, Svedhem et al., 2003, Iwasaki et al., 2003, Willumeit et al., 2007). Auto- and photooxidized lipids were reported to have negative effects on cells if ingested, as peroxides and photooxidation end products (such as malondialdehyde, 4-hydroxyalkenals) cause oxide radicals which in turn cause damage to DNA and proteins (Refsgaard et al., 2000, Cheung et al., 2007). Epoxides of AA on the other hand, another eicosanoid, have been shown to have important biological functions (Capdevila et al., 2000). EPA, just as AA, is prone to auto- and photooxidation on air due to its unsaturations in the carbon chain; thus the results of the in vitro tests, which showed a positive effect of the coating by irradiation, are of interest and will be further investigated in long term in vitro tests and in vivo studies. In later studies a coating method could be tested where the Ti surfaces alone are activated with UV irradiation and the EPA is given to the surfaces subsequently. By this the alteration of the fatty acid molecules may be reduced, and results compared with the present investigation.

## Example 3

Experiments performed *in vivo* with titanium implants containing a high dosage of physical adsorbed EPA and a low dosage of chemically-bound EPA.

### Materials and methods

### Titanium coins

Commercially pure (cp) machined titanium implants with a diameter of 6.25 mm and a height of 1.95 mm were cleaned and sterilized before use. Briefly, implants were washed together in a glass beaker with deionised water for 30 s, then with 70% ethanol for 30 s, and then with ultrasonic bath at 40°C for 5 min in deionised water. The implants were subsequently placed in 40% NaOH solution in a water bath of 40°C for 10 min, sonicated in deionised water for 5 min, and then washed with deionised water until the pH reached 6. Afterwards the implants were sonicated in deionised water at 50°C for 5 min, placed in 50% $HNO_3$ solution at 50°C for 10 min, and sonicated in deionised water for another 5 min. The implants were washed with deionised water until reached pH=6 and were stored in 70% ethanol. Before use, the coins were rinsed with water, rinsed with ethanol, sonicated for 5 min at room temperature and rinsed with deionised water. The titanium coins were then sterilised by autoclaving at 121 °C for 15 min.

### Preparation of implants

For the surface modification of titanium implants, EPA was dissolved in methanol to 10 mM solution. The EPA solution was filtered with a 0.22 $\mu$m pore size filter to sterilise it before use. Four groups of coins were prepared; non-irradiated and unwashed coins (Control), high dosage of EPA (EPA), UV-irradiated coins (UV) and low dosage of EPA after UV irradiation and washing (UV +EPA). When EPA was given to the surface, 10 $\mu$l of the respective 10 mM EPA solution was used, which equals total 3.2 nmol EPA/mm$^2$. The coins were allowed to dry on air for 15 min in a sterile flow bench (all four groups of coins). Coins of UV and UV-EPA groups were then irradiated with UV light for 30 min (Fluo.link, $\lambda$ = 312 nm, I = 6 mW/cm$^2$). These last groups of coins (UV and UV-EPA groups) were washed afterwards with methanol to remove unbound EPA, and to follow the same procedure for the control coins irradiated with UV and without EPA. The coins were allowed to air-dry for 15 min in the sterile flow bench and packed in sterile microcentrifuge tubes.

### Materials and methods for XPS analysis

Kratos Axis Ultra$^{DLD}$ XPS instrument (Kratos, Manchester, UK) was used to perform surface analysis of the modified implants' surfaces. Monochromatic Al K$\alpha$ x-rays were used with a current of 10 mA and a voltage of 15 kV. An area of 300 x 700 $\mu$m$^2$ was analysed on one sample per each group, with a pass energy of 80.0 eV for survey scans and 20.0 eV for high energy resolution elemental scans of Ti 2p, C 1 s, O 1 s, and N 1 s. The peak areas were calculated with the program CasaXPS 3.2.12. The binding energy scale was calibrated by assigning the hydrocarbon peak to a binding energy of 284.8 eV. Shirley background was used to quantify the survey spectra, and mixed Shirley and linear backgrounds were used for the quantification of the detailed elemental scans.

**Animal study and pull-out analysis**

Six New Zealand White female rabbits, 6 months old and a weight of 3.0-3.5 kg, were used in this study (ESF ProdukterEstuna AB, Norrtälje, Sweden).

The implants with high dosage of EPA (n=6) and their controls (n=6), and implants with a low dosage of EPA chemically bound after UV light irradiation (n=6) and their UV-irradiated controls (n=6) were placed in calibrated cortical bone defects in the tibia of rabbits (New Zealand White). The methods used were all according to a standardized and validated model established for the study of bone attachment to titanium implant surfaces (Rønold and Ellingsen, 2002). Each rabbit received four implants, two in each tibia bone. Location of test and control implants was randomized and the operator was blinded. At 10 weeks after implantation the rabbits were sacrificed and the tibia bones with the implants attached were excised. Directly after excising the tibia bone was fixed in a specially designed jig, and the implants were detached using a calibrated pull-out procedure. The load was applied until loosening of the implant and recorded on a load versus time plot. The remaining tibial bone was used for studying volumetric bone mineral density (vBMD) using micro-computed tomography (micro-CT). Wound fluid was collected from the implant site after removal of implants for LDH, ALP activity and total protein. The implants were directly transferred to a sterile tube and processed for RNA extraction, for gene expression analysis of inflammatory and bone markers.

**Micro-tomography analysis**

The tibia was fixated in 4% neutral buffered formaldehyde and then kept in 70 % ethanol. Bone samples were scanned with a commercially available desktop micro-CT scanner. Bone mineral density was studied in the sub-implant cortical bone after implant removal, to study changes in mineralization in the peri-implant bone, from new bone (lower in vBMD) to more mature bone (higher in vBMD).

**Wound fluid analyses: LDH activity, ALP activity and total protein**

LDH activity and total protein was analysed in the wound fluid collected from the implant site following a 10 week healing period. The release of LDH is a sensitive marker for tissue necrosis (Williams et al., 1983) and thus the biocompatibility of the implants. ALP activity has been proposed to play an important role in the initiation of mineralization process around titanium implants. The amount of total protein was used to indicate the amount of wound fluid present at the interface with the implants, and to correlate with the bone-to-implant attachment strength.

**In vivo gene expression of inflammatory and bone markers: IL-6, osteocalcin and TRAP**

Gene expression of IL-6, osteocalcin and TRAP was studied using real-time RT-PCR as an indication of inflammation (IL-6) bone formation (osteocalcin) or bone resorption (TRAP) in the peri-implant bone tissue attached to the surface of the different groups of implants.

**Conclusion**

The elemental compositions of the surfaces were calculated from the survey spectra of the respective sample and are given in Table 3. The C content increased with addition of EPA to the surfaces, while the Ti signals decreased for those samples, indicating that a thin layer of EPA was left on the surfaces after washing. The survey showed as well a clear reduction of N signal after UV irradiation. N and various contaminants such as Na, Zn, Mg, and Si were detected in traces, probably due to surface contaminations from air. The detail spectra revealed further details about the binding states of elements (for C1s see Table 4 and for O1s see Table 5). Up to 4 peaks (3 for Control, UV, and EPA) were fitted into the detail spectra of C1 s. C-C, and C-H signals were detected at a BE (binding energy) of 284.8 eV (Han *et al.*, 2008; Viornery *et al.*, 2002). The intensity of this signal decreased slightly for the EPA surface, and more clearly for the surface UV + EPA. The intensity of the C-O signal at BE = 285.8 eV (Han *et al.*, 2008; Viornery *et al.*, 2002) remained approximately similar for all analysed surfaces. A signal for C=O binding states at 287.9 eV [22] was only detected for the surfaces of the group UV + EPA. The signal assigned to -O-C=O at 288.8 eV (Han *et al.*, 2008; Viornery *et al.*, 2002) was low on control surfaces, increased for all the other surfaces, and highest for UV + EPA surfaces. Up to 5 peaks (4 peaks for Control and UV) were fitted in the detail spectra of O1s. The $TiO_2$ signal was assigned to BE = 529.9 eV (Viornery *et al.*, 2002; Lu *et al.*, 2000). It was decreased for the EPA surface and even lower for the UV + EPA surface. TiO and TiOH gave a signal at the same BE; 531.2 eV (Viornery *et al.*, 2002; Lu *et al.*, 2000). It was slightly increased for the surface of the UV group and about 2-fold increased for the surface of the UV + EPA group. The percentage of the peak for C-O, C=O, and C-OH at 532.4 eV (Viornery *et al.*, 2002) was slightly decreased on UV surfaces compared to Control, increased on EPA surfaces, and decreased on UV + EPA surfaces compared to Control. O that was bound in combinations containing -O-C=O was fitted at 533.7 (Beamson *et al.,* 1992). This variation was present only in surfaces of EPA and UV + EPA.

Table 3: Results of elemental analysis from XPS survey spectra on the differently modified implant surfaces.

| Sample | Element composition [at.%] | | | | |
|---|---|---|---|---|---|
| | C | O | Ti | N | Contaminants |
| Control | 52,2 | 31,8 | 12,6 | 1,4 | 2,1 |
| EPA | 60,4 | 27,4 | 7,2 | 1 | 3,8 |
| UV | 46,5 | 35,2 | 13,5 | ND | 4,8 |
| UV + EPA | 62,6 | 28,3 | 6,3 | ND | 2,8 |
| ND - Not detected | | | | | |

Table 4: Percentage of area for the peaks fitted into the detail spectra of the C1s speak.

| Sample | Percentage areas of the deconvoluted peaks | | | | |
|---|---|---|---|---|---|
| | Binding Energy [eV] | 284,8 eV | 285,8 eV | 287,9 eV | 288,8 eV |
| | Possible bonding states | C-C / C-H | C-O | C=O | -O-C=O |
| Control | | 71,3 | 26,5 | ND | 2,2 |
| EPA | | 69,2 | 25,8 | ND | 5,0 |
| UV | | 71,4 | 24,3 | ND | 4,3 |
| UV+EPA | | 66,8 | 25,0 | 2,9 | 5,4 |

Table 5: Percentage of area for the peaks fitted into the detail spectra of the O1s peak.

| Sample | Percentage areas of the deconvoluted peaks | | | | |
|---|---|---|---|---|---|
| | Binding Energy [eV] | 529,8 eV | 531,2 eV | 532,4 eV | 533,8 eV |
| | Possible bonding states | TiO2 | TiO / TiOH | C-O / C=O / C-OH | -O-C=O |
| Control | 61,7 | 23,1 | 15,2 | ND | |
| EPA | 47,4 | 19,5 | 26,0 | 7,1 | |
| UV | 59,0 | 28,1 | 12,9 | ND | |
| UV+EPA | 36,8 | 47,4 | 7,9 | 7,8 | |

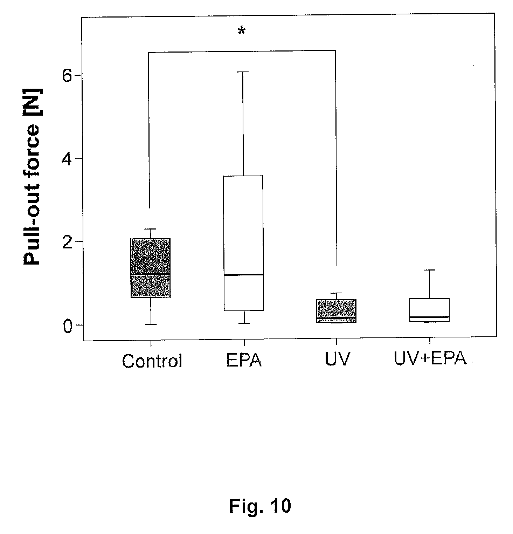

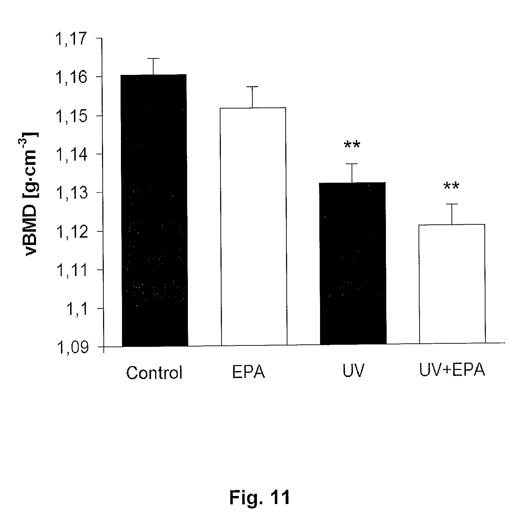

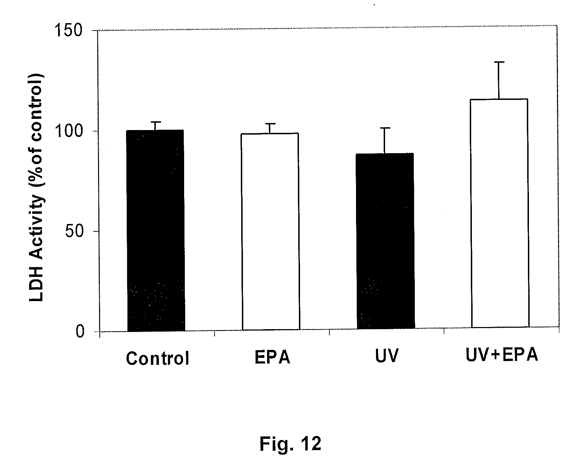

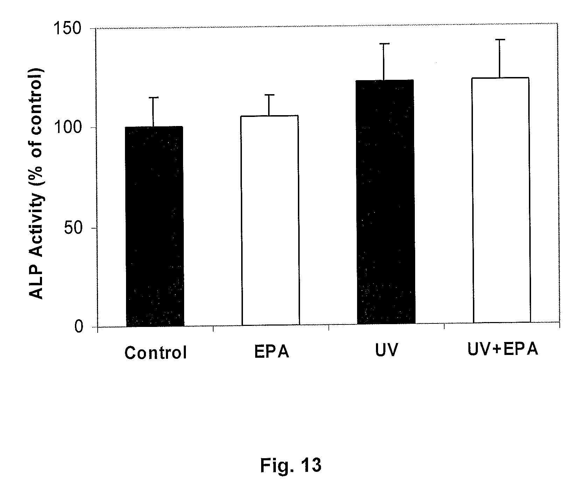

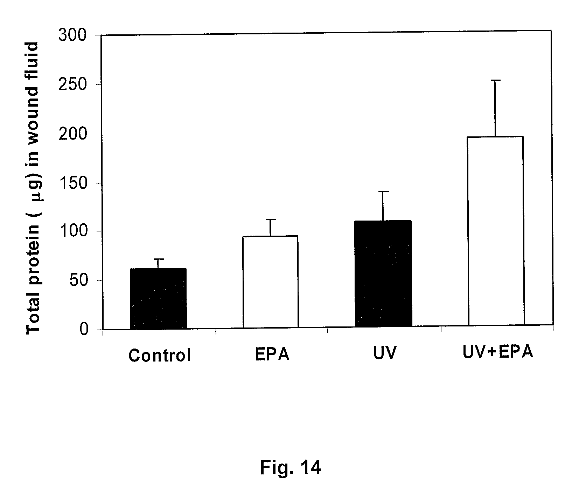

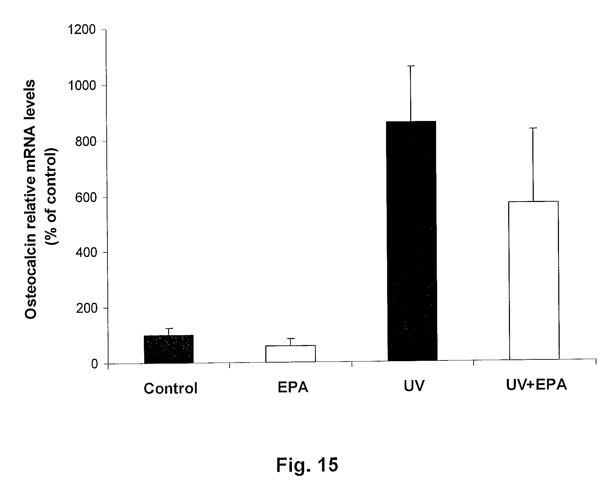

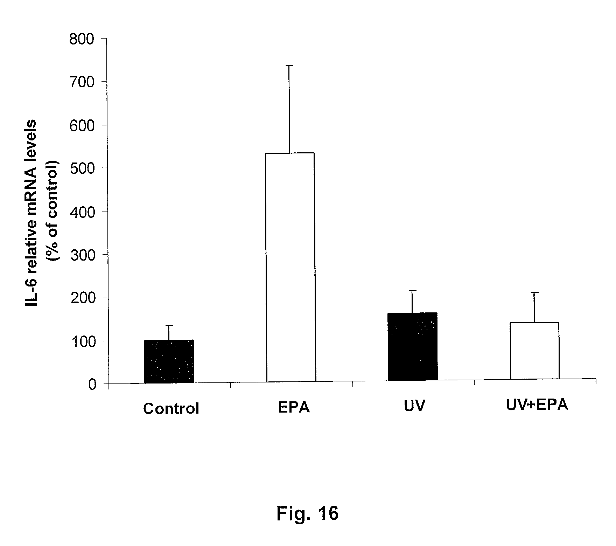

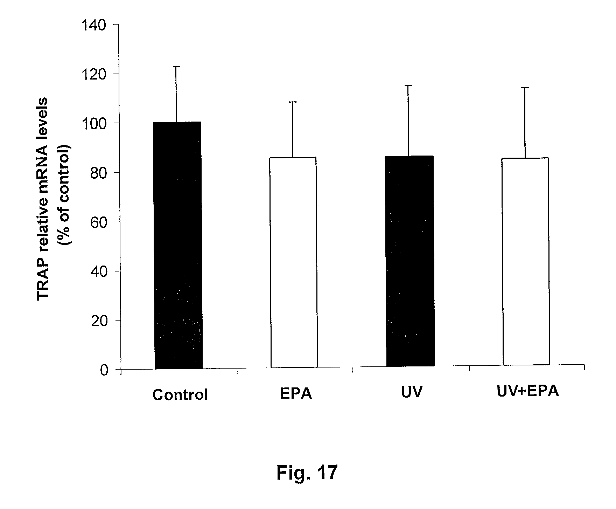

The implants coated with a low dosage of EPA chemically bound after UV light irradiation and their UV-irradiated controls showed a lower attachment to the cortical bone. A lower vBMD of the cortical bone in both type of implants was observed, which could be related with the differences in the biomechanical properties of peri-implant cortical bone found with the pull-out values. The amount of total protein in the wound fluid, higher in the implants coated with a low dosage of EPA chemically bound after UV light irradiation and their UV-irradiated controls, could also be related with their lower pull-out values. No differences in LDH activity (biocompatibility) were observed with the four different groups of implants, but an increase in the gene expression of the inflammatory marker IL-6 was observed in the group of implants with high dosages of EPA. No differences were found in the gene expression of TRAP, suggesting the bone resorption was not affected differently with the implants tested. The implants coated with a low dosage of EPA chemically bound after UV light irradiation and their UV-irradiated controls showed a higher expression of the pro-osteogenic marker osteocalcin and a higher ALP activity, indicating that osteoblastic cell activity and on-going bone formation was improved in these samples. The results of this study are shown in Figures 10-17.

**Example 4**

Titanium (Ti) surfaces were coated with two different n-3 polyunsaturated fatty acids (PUFAs), without and in combination with $\alpha$-tocopherol (vitamin E) to analyse the antioxidative properties of $\alpha$-tocopherol on PUFAs during UV irradiation. $\alpha$-tocopherol is a substance that is considered safe by the authorities as a food additive. $\alpha$-tocopherol has

been shown to have anti-inflammatory effects *in vitro* by inhibiting IL-1β release from monocytes. At the same time, a decrease in cell adhesion was observed (Reno et al, 2005). We hypothesized that coating of Ti surfaces with PUFAs in combination with α-tocopherol could prevent oxidation of PUFA and result in a Ti surface with improved properties.

**Materials and Methods**

The coating substances were purchased from Sigma-Aldrich with the highest grade of purity available. Surfaces of Ti (c.p. grade IV) disks, 6.25 mm in diameter, were coated with different substances and mixtures thereof. The surfaces were dried on air and subsequently irradiated with UV light (λ=302 nm, P=6 W, distance to surfaces 43 mm, lamp purchased from VWR, Oslo, Norway). The samples were analysed with FTIR spectroscopy (DRIFT) after 0 min, 15 min, 30 min, and 60 min of irradiation. An equally irradiated, uncoated Ti disk was used as a background for the FTIR measurements. Mixtures were applied to the implants and irradiated for 0 min, 15 min, 30 min, and 60 min. The spectra obtained by FTIR spectroscopy were analysed for typical absorbances connected with photooxidation of the surface coatings. Typical peak areas were quantified and will be compared and discussed in this document.

The substances used as coating were:

EPA (eicosapentaenoic acid; 20:5, n-3); 10 μl of 50 mM solution in ethanol
DHA (docosahexaenoic acid; 22:6, n-3); 10 μl of 50 mM solution in ethanol
EPA + DHA; 1:1 - 10 μl in total
α-tocopherol; 10 μl of 50 mM solution in ethanol
EPA + α-tocopherol; 1:1 - 10 μl in total
DHA + α-tocopherol; 1:1 - 10 μl in total
EPA + DHA + α-tocopherol; 1:1:1 - 10 μl in total

The presentation of the results of the analysis is organised as follows:

1. UV irradiation of the n-3 PUFAs EPA, DHA, and EPA + DHA
2. UV irradiation of α-tocopherol
3. Combination of α-tocopherol and the n-3 PUFAs

1. UV irradiation of the n-3 PUFAs EPA, DHA, and EPA + DHA

The most important absorbances that showed changes in the n-3 PUFA chemical structure due to UV irradiation are given in the following table (Table 6):

Table 6: Important changes of absorbances in FTIR spectroscopy for PUFAs

| Wavenumber (max absorbance) | Group | Changes caused by UV irradiation |
|---|---|---|
| 3300 cm-1 | -OH | increase |
| 1710 cm-1 | C=O | increase |
| 1065 cm-1 | CO-O-C | change |
| 974 cm-1 | C=C trans | appearance and increase |
| 710 cm-1 | C=C cis | decrease |

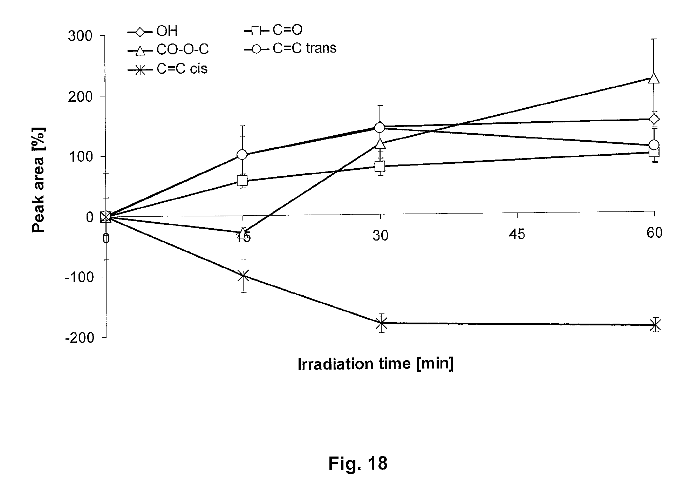

Figure 18 shows how the mentioned absorbencies are changing. The values shown are calculated from the absorbance peak areas and are given as % compared to non-irradiated sample. Very interesting are the opposing trends of the absorbances of C=C cis and C=C trans, which indicate the formation of trans fatty acids from cis fatty acids. Oxidation caused by UV irradiation was observed.

2. UV irradiation of α-tocopherol

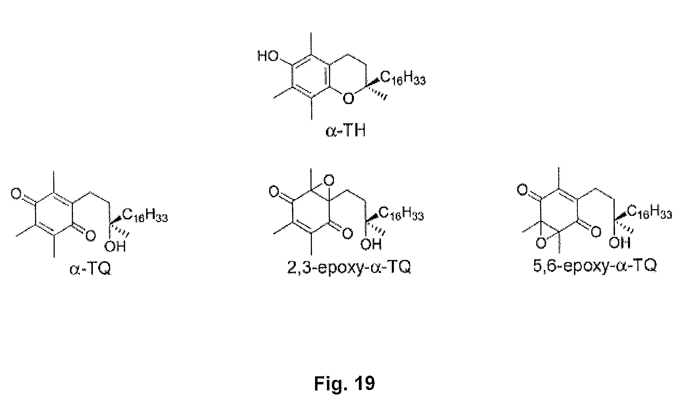

The Chemistry of the oxidation process of α-tocopherol was described by Wang X, 1999: TOH → TO˙ (α-tocopherol → α-tocopheroxyloxyl) and Yamauchi R, 2002 (see Fig. 19):
According to those sources, the following absorbances are of interest as they might be changing during UV irradiation (Table 7):

Table 7: Important changes of absorbances in FTIR spectroscopy for α-tocopherol

| Wavenumber (max absorbance) | Group | Changes caused by UV irradiation |
| --- | --- | --- |
| 1676 cm-1, 1646 cm-1 | Quinones | appearance/ increase |
| 1620 cm-1 | C=C aromatic | increase |
| 1250 cm-1 | C - O of epoxides | appearance/ increase |

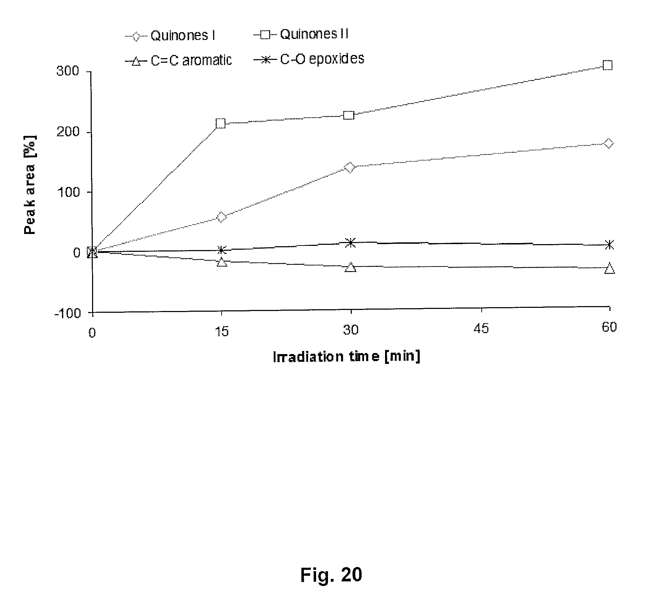

As can be seen from Figure 20, the absorbances of quinones were increased during UV irradiation while the absorbance areas for epoxides did not change, indicating that no epoxides were formed from α-tocopherol during UV irradiation, but quinones, such as α-tocopherylquinone. The absorbance of aromatic C=C groups decreased during UV irradiation, indicating that those bindings were reduced by photooxidation.

3. Combination of α-tocopherol and the n-3 PUFAs

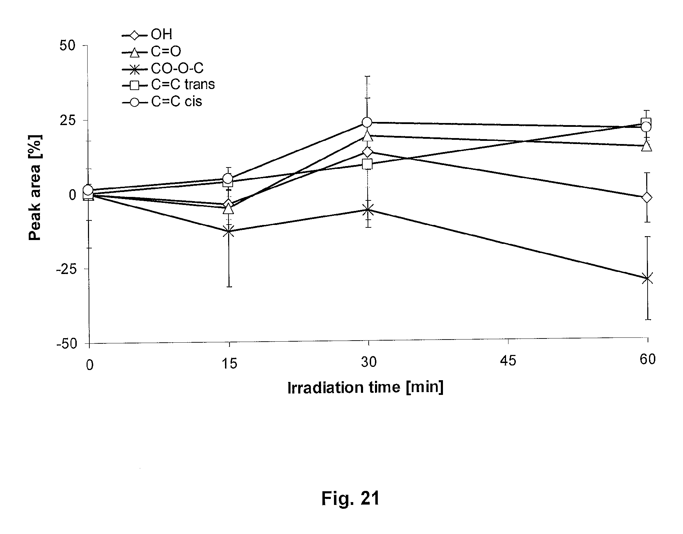

Addition of α-tocopherol to EPA, DHA, and EPA+DHA was tested. Figure 21 shows the changes of peak areas of the characteristic absorbances for the averages of peak areas of EPA and DHA with α-tocopherol added. The changes of peak areas with UV irradiation time were very slight compared with changes of peak areas for the PUFAs alone, indicating a protective effect of α-tocopherol.

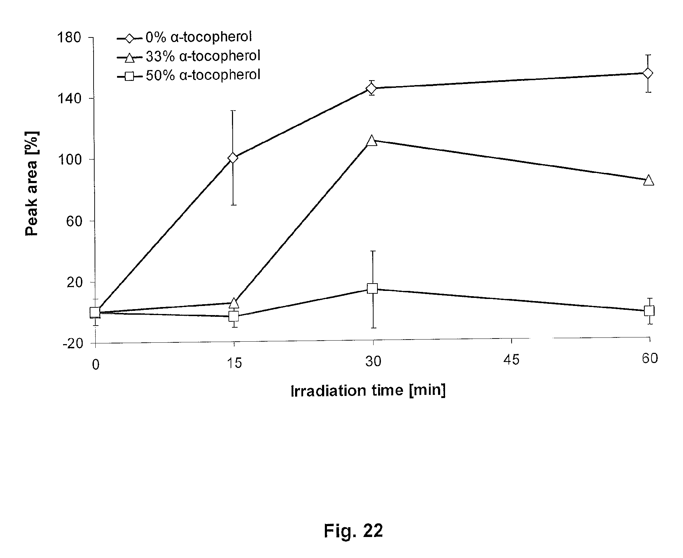

The antioxidative effect of α-tocopherol was dependent on the amount of α-tocopherol added, as could be shown for instance on the example of OH absorbances at 3300 cm-1 (Figure 22). The increase of peak area was much more pronounced for the PUFAs alone compared to 1:1 mixtures with 50% α-tocopherol (EPA+α-tocopherol, DHA+α-tocopherol) or 1:1:1 mixtures (EPA+DHA+α-tocopherol, 33 mol% α-tocopherol).

**Conclusion**

UV irradiation was shown to change the molecular structure of photosensitive PUFAs such as EPA and DHA in a similar manner. As for a typical photooxidation process, the absorbance intensities increased for OH, CO-O-C, C=O, and C=C trans groups, while the absorbance intensity decreased for the C=C cis group. α-tocopherol seemed to be less affected by UV irradiation up to 1 h, only the appearance of a weak quinone absorbance was observed. This is in accordance to one oxidation pathway proposed by Yamauchi et al. Addition of α-tocopherol to the PUFAs in 2 different concentrations indicated a concentration dependent protective effect of the α-tocopherol.

**Example 5**

7-Dehydrocholesterol (7-DHC, provitamin $D_3$) is converted to cholecalciferol (vitamin $D_3$) via previtamin $D_3$ in the human skin. Further conversion to $25(OH)D_3$ is mainly reported to happen in the liver, but since the hydroxylase enzyme CYP27A1 is ubiquitously expressed in other tissues, the system for local productions is also available in bone (Aiba I et al., 2006). $1\alpha,25(OH)_2D_3$ has as well been shown to be synthesised locally in bone from $25(OH)D_3$ (Ichikawa et al., 1995; Atkins et al., 2007). A schematic overview of the conversion can be found as Figure 23. $1\alpha,25(OH)_2D_3$ is the biological active form of vitamin $D_3$ and acts as a hormone to regulate serum calcium and phosphate levels and is an important factor during bone growth and mineralisation (St-Arnaud R, 2008). Thus, adding 7-DHC as a surface coating to titanium implants irradiated with UV light, with or without PUFA, might affect bone healing positively.

Titanium (Ti) surfaces were coated with either 7-DHC or cholecalciferol to analyse their transition initiated by UV light. We aimed to specify how specific the UV initiated conversion from 7-DHC to cholecalciferol was and which irradiation time would be the most appropriate. Furthermore, the surfaces were coated with a mixture of EPA

(representing an n-3 PUFA) and 7-DHC to analyse possible interactions between the 2 substances during UV irradiation.

**Materials and Methods:**

EPA, 7-DHC and cholecalciferol were purchased from Sigma-Aldrich with the highest grade of purity available. Surfaces of Ti (c.p. grade IV) disks, 6.25 mm in diameter, were coated with either 7-DHC or cholecalciferol, or a mixture of 7-DHC + EPA (1:1).The surfaces were dried on air and subsequently irradiated with UV light (λ=302 nm, P=6 W, distance to surfaces 43 mm, lamp purchased from VWR, Oslo, Norway). The samples were analysed with FTIR spectroscopy (DRIFT) after 0 min, 15 min, 30 min, and 60 min of irradiation. An equally irradiated, uncoated Ti disk was used as a background for the FTIR measurements. The spectra obtained by FTIR spectroscopy were analysed for typical absorbances connected with photooxidation of the surface coatings. Typical peak areas were quantified if possible and will be compared and discussed in this document.

**Results**

The most important absorbances that showed changes in the chemical structure of 7-DHC and cholecalciferol due to UV irradiation are given in the following table (Table 8):

Table 8: Important changes of absorbances in FTIR spectroscopy for 7-DHC and cholecalciferol

| Wavenumber (max absorbance) | Group | Changes caused by UV irradiation of | |
|---|---|---|---|
| | | 7-DHC and cholecalciferol | 7-DHC + EPA |
| 3300 cm-1 | -OH | no changes | increase |
| 1730 cm-1 | C=O ester groups | increase | |
| 1710 cm-1 | C=O carboxylic acids | increase | |
| 1680 cm-1 | C=C trans | appearance and increase | |
| 1650 cm-1 | C=C cis | increase | |
| 1625 cm-1 | C=C aromatic | increase | |

*a) UV irradiation of 7-DHC and cholecalciferol*

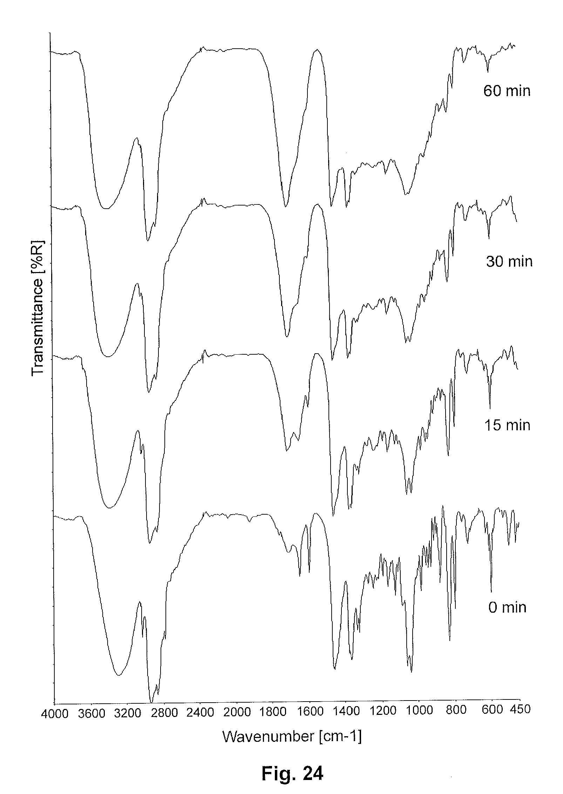

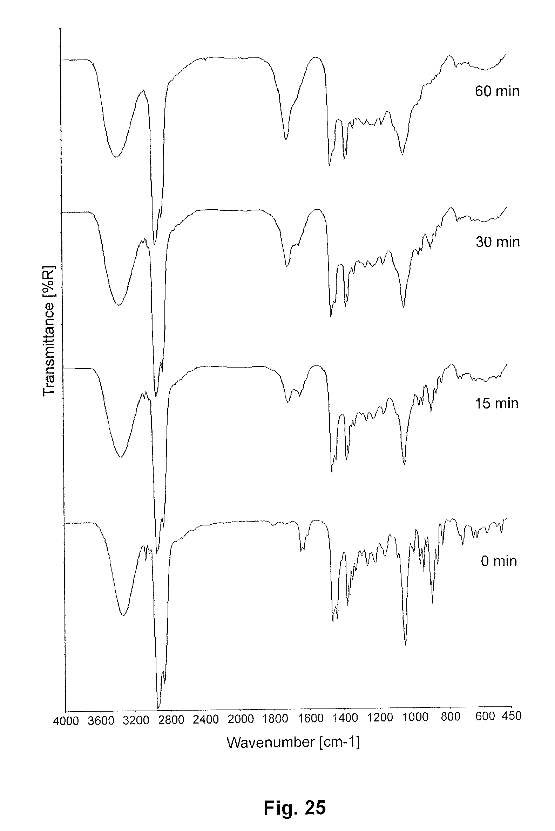

The following Figures 24 and 25 show how the absorbance spectra of 7-DHC and cholecalciferol were changing with UV irradiation time. As the chemical structures of the 2 substances are very alike, the absorbance spectra appeared to be quite similar. Also their behaviour with UV irradiation time appeared to be comparable (Table 8).

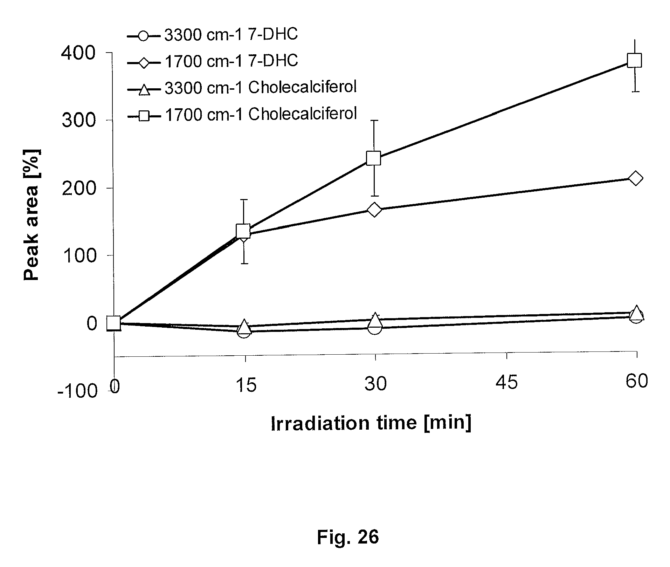

From the absorbance spectra and the changes of peak areas measured (Figure 26), we can assume that no -OH groups were generated due to the irradiation. The peak area at 3300 $cm^{-1}$ was stable with UV irradiation time. The peak area at about 1700 $cm^{-1}$ (including the area from 1850 $cm^{-1}$ to 1550 $cm^{-1}$) was increasing clearly for both substances, indicating the formation of C=C double bonds (aromatic, cis- and trans-), as well as the formation of C=O ester and carboxylic groups.

*b) UV irradiation of 7-DHC + EPA*

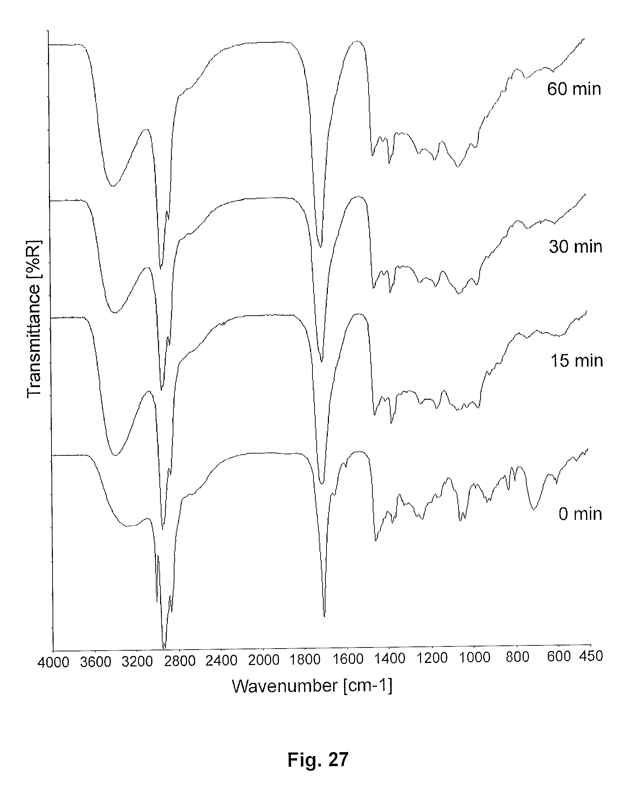

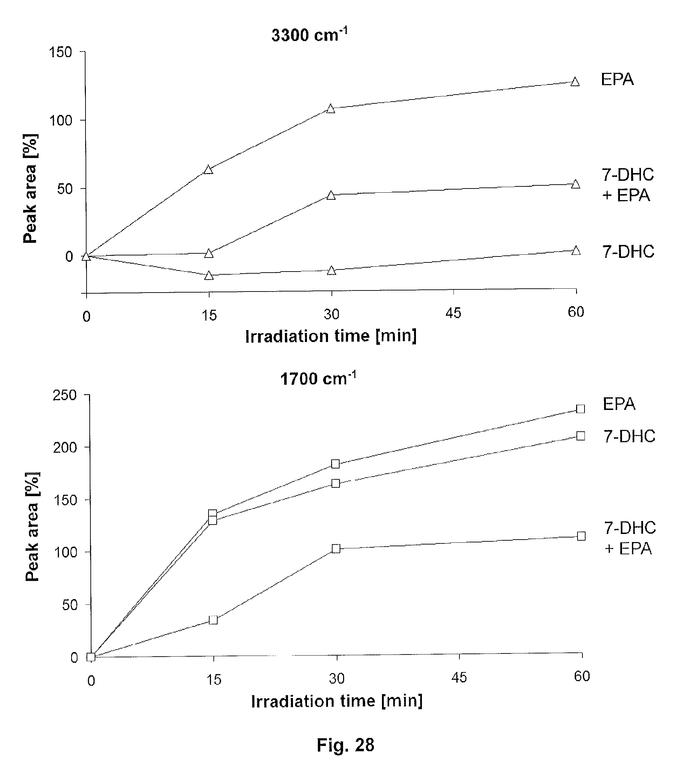

The spectra of the mixture of 7-DHC and EPA seemed to represent both substances in their typical absorbances (Figure 27). The changes of the peak areas (Figure 28) confirm this observation. At 3300 $cm^{-1}$ we found an increase in peak area that lies in between the values for 7-DHC (no changes) and the values for EPA (clear increase). At 1700 $cm^{-1}$ we found the same result, which only becomes clear in the absolute values which again are in between the values measured for 7-DHC (slow increase from small peak area) and EPA (steep increase from large peak area).

**Conclusions**

None of the absorbance spectra of 7-DHC after UV irradiation was completely similar to the absorbance spectrum of non-irradiated cholecalciferol. A likely explanation for this is that a mixture of several photooxidation products is formed due to UV irradiation as described in the literature (see Figure 23). The conversion from 7-DHC to cholecalciferol is about 0.8 % only (Olds et al., 2008) and thus may not be detectable with FTIR. This is not a problem, since all the photooxidation products produced by UV irradiation also evolve in the human body and are biological inactive, thus preventing hypervitaminosis of vitamin $D_3$.

Combining equal amounts of 7-DHC and EPA on Ti surfaces did not result in any chemical interactions between the 2 substances that could be measured with FTIR spectroscopy. The irradiation of the mixture resulted in spectra that had the appearance and absorbance peak areas that were a combination from the spectra and peak areas of 7-DHC and EPA. Using other PUFAs than EPA is not expected to result in findings different from this.

**Example 6**

This example demonstrates how smooth and rough implants with PUFA and/or vitamins may be manufactured.

**A.1. Smooth implants:**

Commercially pure (cp) titanium disks with a diameter of 6.25 mm and a height of 2 mm were grinded and polished (Phoenix 4000, Buehler GmbH, Duesseldorf, Germany) in seven sequences. The silicon carbide papers, the porous neoprene for final polishing and the abrasive colloidal silica suspension (OP-S) were supplied by Struers GmbH (Willich, Germany). The first step consisted in grinding all the implants with P500 in water until they were levelled, with 65 N of pressure and a contra-rotation at 250 rpm. Then, the grinding time was set down to 20 min and the grain size decreased with papers: P800; P1200 and P2500. For the P4000 polishing paper, the OP-S polishing suspension was used. For the last two steps, a special porous neoprene (MD-Chem) was used at 200 rpm and 50 N of pressure, 14 min in co-rotation with OP-S suspension, and then 1 min in co-rotation with water. The grinding/polishing were necessary to create uniform plane and clean surfaces prior to modification. After polishing, all the disks were washed alternately with NaOH at 40 vol. % and HNO3 at 50 vol. % in ultrasonic bath. Then washed with deionised water to reach a neutral pH, and stored at room temperature in 70 vol. % ethanol.

### A. 2. Rough implants:

Commercially pure (cp) titanium disks with a diameter of 6.25 mm and a height of 2 mm were blasted with titanium dioxide ($TiO_2$) particles with a particle size of 180-220 $\mu$m (Blasmaster™ oxide powder, F.J. Brodmann&CO, L.L.C., Los Angeles, USA) after polishing. The distance from the implants to the jets was approximately 20 mm during blasting procedure and the TiO2 particle stream hit the surface with an angle of 90°. The air pressure used for blasting was set to 0.5 Mpa. After polishing and blasting, all the disks were washed alternately with NaOH at 40 vol. % and HNO3 at 50 vol. % in ultrasonic bath. Then washed with deionised water to reach a neutral pH, and stored at room temperature in 70 vol. % ethanol.

### B. Surface modification of smooth and rough implants with PUFA and/or vitamins

For the surface modification of smooth and rough implants with PUFA and/or vitamins:

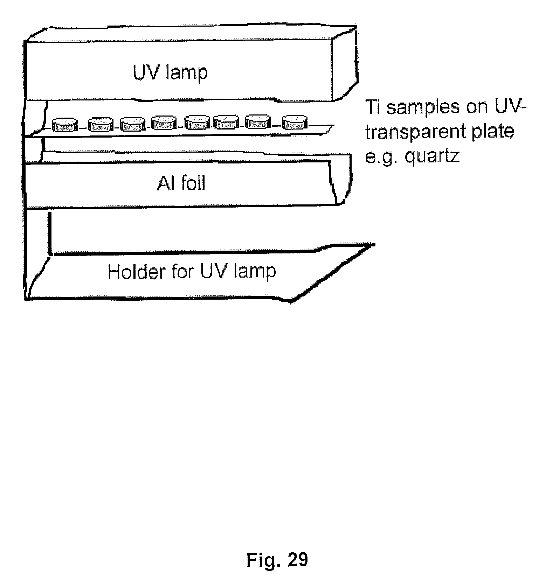

1. Work in sterile bench.
2. Solutions of the respective substances are prepared with ethanol.
3. Working solutions are filtered (0.2 $\mu$m pore size) to sterilise them.
4. 10 $\mu$l of respective solution is given to the surface of the coins (with the selected concentration and combination of PUFA/vitamins).
5. Coins are allowed to dry on sterile bench for 15 min.
6. The samples are irradiated with UV light (UV C 100 nm - 280 nm, and UV B light 280 nm - 315 nm). The set-up is given in Figure 29. One problem is to irradiate the samples from all sides. A reflecting Al foil can be bended and used to access the samples from down and the sides if the samples are placed on a UV transparent surface (e.g. quartz). Figure 29 shows an examplary set-up for irradiating Ti samples with UV light.

### D. Characterization of smooth and rough implants modified with PUFA and/or vitamins:

1. Profilometer: a blue light laser profilometer (Sensofar Plµ 2300, Terrassa, Spain) is utilised to scan 200 x 160 $\mu$m2 areas with a 150x (150x/0.95 Lu Plan Apo, EPI) Nikon (Nikon, Tokyo, Japan) objective. The roughness and waviness parameters from each coin were then calculated with advanced software (SensoMap Plus 4.1, Sensofar, Terrassa, Spain), and representative pictures of the different surfaces are chosen.
2. Contact angle: measured at least 24 h after end of UV irradiation. The contact angles (CA) of the surfaces of the different titanium implants are analyzed using a video-based contact angle system (OCA 20, DataPhysics Instruments GmbH, Filderstadt, Germany). The fluid used was deionised water. A drop of 3 $\mu$l was given to the surface with a velocity of 0.6 $\mu$l s-1. The drop shape is recorded with a camera (4.17 pictures s-1) and contact angles were measured afterwards from the recorded pictures. A drop of 3 $\mu$l on coin, CA is measured, the drop is enlarged 2x with 1 $\mu$l of pure water, after each enlargement the surface CA is measured, the CA is not measured after drying the coin as this could change the structure of the PUFA on the surface of the coin and therewith the CA.
3. FT IR (diffuse reflectance) to characterise the difference before and after UV irradiation of PUFA and vitamins. Films of PUFA and/or and the changes caused by UV irradiation were analysed with FTIR Spectrum 100, Perkin Elmer (USA) The diffuse reflectance unit (DRIFT) is used to collect the spectra. A cleaned, sterilised, but unmodified polished Ti coin served as reference. The FTIR spectra is collected before and after irradiation with UV light at different time points of irradiation. The spectra were collected from 4000 cm-1 to 450 cm-1 with a resolution of 2 cm-1. Each spectrum was the result of 8 single spectra. The spectra were baseline corrected with the program Spectrum (Version 6.1.0) from PerkinElmer.

4. FTIR-microscopy is performed to answer the following questions:

- How are the components distributed before and after UV irradiation?
- Is there a difference if the components are alone on the surface or in different combinations?
- How is the crystalline structure of the Ti coins?
- Is there any correlation between the distribution of components and the crystals?

5. XPS is performed to get information about the atomic composition of the surfaces. Kratos Axis UltraDLD XPS instrument (Kratos, Manchester, UK) is used to perform surface analysis of the modified implants' surfaces. Monochromatic Al K$\alpha$ x-rays were used with a current of 10 mA and a voltage of 15 kV. An area of 300 x 700 $\mu$m2 was analysed on one sample per each group, with a pass energy of 80.0 eV for survey scans and 20.0 eV for high energy resolution elemental scans of Ti 2p, C 1 s, O 1 s, and N 1 s. The peak areas were calculated with the program CasaXPS 3.2.12. The binding energy scale was calibrated by assigning the hydrocarbon peak to a binding energy of 284.8 eV. Shirley background was used to quantify the survey spectra, and mixed Shirley and linear backgrounds were used for the quantification of the detailed elemental scans.

**References**

Guillén MD, Cabo N. Infrared Spectroscopy in the Study of Edible Oils and Fats. Journal of the Science of Food and Agriculture 1997; 75:1-11.

Kim HK, Kim K, Byun Y. Preparation of a chemically anchored phospholipid monolayer on an acrylated polymer substrate. Biomaterials 2005; 26(17):3435-3444.

Nakamura M, Sirghi L, Aoki T, Hatanaka Y. Study on hydrophilic property of hydro-oxygenated amorphous TiOx:OH thin films. Surface Science 2002; 507-510:778-782.

Rønold HJ, Ellingsen JE. The use of a coin shaped implant for direct in situ measurement of attachment strength for osseointegrating biomaterial surfaces. Biomaterials 2002; 23(10):2201-2209.

Satsangi N, Satsangi A, Glover R, Ong JL, Satsangi RK. Osteoblast response and calcium deposition on phospholipid modified surfaces. J Mater Sci Mater Med 2004; 15(6):693-7.

Miyauchi M, Watanabe T, Hashimoto K, Kieda N, Hishita S, Mitsuhashi T, Nakajima A. Reversible wettability control of TiO2 surface by light irradiation. Surface Science 2002; 511:401-407.

Zhao G, Schwartz Z, Wieland M, Geis-Gerstorfer J, Cochran DL, Boyan BD. High surface energy enhances cell response to titanium substrate microstructure. Journal of Biomedical Materials Research A 2005; 74A(1):49-58.

Wang R, Hashimoto K, Fujishima A, Chikuni M, Kojima E, Kitamura A, Shimohigoshi M, Watanabe T. Photogeneration of Highly Amphiphilic TiO2 Surfaces. Advanced Materials 1998; 10(2):135-138.

Moison RM, Steenvoorden DP, Beijersbergen van Henegouwen GM. Topically applied eicosapentaenoic acid protects against local immunosuppression induced by UVB irradiation, cis-urocanic acid and thymidine dinucleotides. Photochem Photobiol 2001; 73(1):64-70.

Khan W, Kapoor M, Kumar N. Covalent attachment of proteins to functionalized polypyrrole-coated metallic surfaces for improved biocompatibility. Acta Biomaterialia 2007; 3(4):541-549;

Muller R, Abke J, Schnell E, Scharnweber D, Kujat R, Englert C, Taheri D, Nerlich M, Angele P. Influence of surface pretreatment of titanium- and cobalt-based biomaterials on covalent immobilization of fibrillar collagen. Biomaterials 2006; 27(22):4059-4068)

Cooper SL, Tegoulia VA, Rao W, Kalambur AT, Rabolt JF. Surface Properties, Fibrinogen Adsorption, and Cellular Interactions of a Novel Phosphorylcholine-Containing Self-Assembled Monolayer on Gold. Langmuir 2001; 17:4396-4404.

Svedhem S, Dahlborg D, Ekeroth J, Kelly J, Hook F, Gold J. In Situ Peptide-Modified Supported Lipid Bilayers for Controlled Cell Attachment. Langmuir 2003; 19:6730-6736.

Iwasaki Y, Tojo Y, Kurosaki T, Nakabayashi N. Reduced adhesion of blood cells to biodegradable polymers by introducing phosphorylcholine moieties. J Biomed Mater Res A 2003; 65(2):164-169

Brunette DM, Tengvall P, Textor M, Thomson P. Titanium in Medicine. Berlin, Heidelberg, New York: Springer-Verlag; 2001.

Hirani N, Chvedov D, Jones R. Characterization of organic monolayers on the surface of aluminum in the process of thermal treatment. Thin Solid Films 2007;in press.

Socrates G. Infrared and Raman Characteristic Group Frequencies: John Wiley & Sons Ltd; 2001.

Cheng H, Jiang, W., Phillips, F.M., Haydon, R.C., Peng, Y., Zhou, L., Luu, H.H., An, N., Breyer, B., Vanichakarn, P., Szatkowski, J.P., Yoon Park, J., He, T.-C.. Osteogenic Activity of the Fourteen Types of Human Bone Morphogenetic Proteins (BMPs). The Journal of Bone and Joint Surgery 2003; 85:1544-1552.

Melø TB, Mahmoud GS. Radicals Induced by Illumination of a Mixture of Unsaturated Fatty Acids with Ultraviolet Light at 77K. Magnetic Resonance in Chemistry 1988; 26:947-954.

Chacon JN, Gaggini P, Sinclair RS, Smith FJ. Photo- and thermal-oxidation studies on methyl and phenyl linoleate: anti-oxidant behaviour and rates of reaction. Chemistry and Physics of Lipids 2000; 107(1):107-120.

Rontani JF. Photodegradation of unsaturated fatty acids in senescent cells of phytoplankton: photoproduct structural identification and mechanistic aspects. Journal of Photochemistry and Photobiology A: Chemistry 1998; 114(1):37-44.

Refsgaard HH, Tsai L, Stadtman ER. Modifications of proteins by polyunsaturated fatty acid peroxidation products. Proc Natl Acad Sci U S A 2000; 97(2):611-616.

Kim B-H, Lee J-Y, Choa Y-H, Higuchi M, Mizutani N. Preparation of TiO2 thin film by liquid sprayed mist CVD method. Materials Science and Engineering B 2004; 107(3):289-294.

Willumeit R, Schuster A, Iliev P, Linser S, Feyerabend F. Phospholipids as implant coatings. J Mater Sci Mater Med 2007; 18(2):367-380.

Cheung SC, Szeto YT, Benzie IF. Antioxidant protection of edible oils. Plant Foods Hum Nutr 2007; 62(1):39-42.

Capdevila JH, Falck JR, Harris RC. Cytochrome P450 and arachidonic acid bioactivation: molecular and functional properties of the arachidonate monooxygenase. Volume 41; 2000. p 163-18.

Williams DL, Marks V. Biochemistry in Clinical Practice. London: William Heineman Medical Books; 1983.

Han, Y, Chen, D, Sun, J, Zhang, Y, Xu, K. UV-enhanced bioactivity and cell response of micro-arc oxidized titania coatings. Acta Biomater 2008;4:1518-1529.

Viornery, C et al. Surface Modification of Titanium with Phosphonic Acid To Improve Bone Bonding: Characterization by XPS and ToF-SIMS. vol. 18, 2002. 2582-2589.

Lu, G, Bernasek, SL, Schwartz, J. Oxidation of a polycrystalline titanium surface by oxygen and water. Surface Science 2000;458:80-90.

Beamson, G, Briggs, D. High resolution XPS of organic polymers : the Scienta ESCA300 database by New York: John Wiley & Sons, 1992.

Reno F, Aina V, Gattia S, Cannas M, Effect of vitamin E addition to poly(D,L) lactic acid on surface properties and osteoblast behaviour. Biomaterials 26 (2005) 5594-5599.

Wang X, Quinn PJ, Vitamin E and its function in membranes. Progress in Lipid Research 38 (1999) 309-336.

Yamauchi R, Noro H, Shimoyamada M, Kato K, Analysis of Vitamin E and Its Oxidation Products by HPLC with Electrochemical Detection. Lipids 37(5) (2002) 515-522.

Aiba I, Yamasaki T, Shinki T, Izumi S, Yamamoto K, Yamada S, Terato H, Ide H, Ohyama Y, Characterization of rat and human CYP2J enzymes as Vitamin D 25-hydroxylases. Steroids 71, 849-856, 2006.

Atkins GJ, Anderson PH, Findlay DM, Welldon KJ, Vincent C, Zannettino ACW, O'Loughlin PD, Morris HA, Metabolism of vitamin D3 in human osteoblasts: Evidence for autocrine and paracrine activities of $1\alpha,25$-dihydroxyvitamin D3. Bone 40, 1517-1528, 2007

Ballard JM, Zhu L, Nelson ED, Seburg RA, Degradation of vitamin D3 in a stressed formulation: The identification of esters of vitamin D3 formed by a transesterification with triglycerides. Journal of Pharmaceutical and Biomedical Analysis 43, 142-150, 2007 Holick MF, Photobiology of Vitamin D in Vitamin D, 2nd Edition (Feldman, Pike, Glorieux), Elsevier 2005.

Holick MF, Photobiology of Vitamin D in Vitamin D, 2nd Edition (Feldman, Pike, Glorieux), Elsevier 2005.

Ichikawa F, Sato K, Nanjo M, Nishii Y, Shinki T, Takahashi N, Suda T, Mouse Primary Osteoblasts Express Vitamin D3, 25-Hydroxylase mRNA and Convert $1\alpha$-Hydroxyvitamin D3, into $1\alpha,25$-Dihydroxyvitamin D3. Bone 16, 129-135, 1995

Olds WJ, McKinley AR, Moore MR, Kimlin MG, In vitro model of vitamin D3 (Cholecalciferol) synthesis by UV radiation: Dose-response relationships. Journal of Photochemistry and Photobiology B: Biology 93, 88-93, 2008

St-Arnaud R, The direct role of vitamin D on bone homeostasis. Archives of Biochemistry and Biophysics 473, 225-230, 2008.

S.-M. Oh et al. 2003 Thin Solid Films 435 (2003) 252-258, page 256.

United States patent US2006088596.

United States patent US2007202149.

United States patent US2008118544.

United States patent US2007299512.

United States patent US2008118544.

## Claims

1. A coated metal implant for controlled adhesion of mineralized and/or hard tissue, wherein at least part of the surface of the implant is coated with chemically UV-bound PUFA (polyunsaturated fatty acids) at a concentration of 10 nanogram/mm$^2$, or less, wherein the coated metal implant is obtainable by a method comprising:

   i) mirror polishing the implant,
   ii) washing,
   iii) autoclaving,

      a) treating the implant with a solution comprising PUFA, and
      b) irradiating at least part of the surface of the implant with UV of 100 to 315 nm for a period of time comprised from 30 seconds to 30 minuts and optionally
      c) washing said implant,

   wherein step a) and b) are performed simultaneously or in any order.

2. The coated metal implant according to claim 1, wherein step b) is performed simultaneously as step a) or alternatively step a) is performed before step b).

3. The coated metal implant according to claims 1 or 2, wherein the UV light of step (b) is approximately 6 mW/cm$^2$.

4. The coated metal implant according to any of the preceding claims, wherein the implant is coated with PUFA at a concentration comprised from 1 to 10 nanogram/mm$^2$.

5. The coated metal implant according to any of the preceding claims, wherein the metal is selected from the group consisting of titanium, titanium alloy, zirconium, hafnium, tantalum, niobium, and mixtures of two or more of zirconium, hafnium, tantalum and niobium.

6. The coated metal implant according to claim 5, wherein the implant comprises at least 90% of weight of titanium and/or an alloy of titanium.

7. The coated metal implant according to any of the preceding claims, wherein the PUFA is selected from the group consisting of n-3 and n-6 fatty acids.

8. The coated metal implant according to any of the preceding claims, wherein the PUFA is selected from the group consisting of eicosapentaenoic acid, arachidonic acid, docosahexaenoic acid and mixtures thereof.

9. The coated metal implant according to any of the preceding claims, wherein the PUFA comprises or is EPA (eicosapentaenoic acid).

10. The coated metal implant according to any of the preceding claims, wherein the implant coating additionally comprises fat-soluble vitamins, such as vitamin E.

11. The coated metal implant according to any of the preceding claims, wherein the implant additionally comprises 7-dehydrocholesterol.

12. The coated metal implant according to any of the preceding claims, wherein when the implant comprises at least 90% of weight of titanium and/or an alloy of titanium, the PUFA comprises or is EPA (eicosapentaenoic acid)., then the coating comprises from 1 to 5x10$^{15}$ bound unsaturated bindings of EPA per mm$^2$ titanium surface.

**Patentansprüche**

1. Ein beschichtetes Metallimplantat zur kontrollierten Adhäsion von mineralisiertem und/oder hartem Gewebe, wobei mindestens ein Teil der Oberfläche des Implantats mit chemisch UV-gebundenen PUFA (polyungesättigten Fettsäuren) bei einer Konzentration von 10 Nanogramm/mm$^2$, oder geringer, beschichtet ist, wobei das beschichtete Metallimplantat durch ein Verfahren erhältlich ist, welches folgendes umfasst:

    i) Hochglanzpolieren des Implantats,
    ii) Waschen,
    iii) Autoklavieren,

        a) das Behandeln des Implantats mit einer PUFA enthaltenden Lösung, und
        b) das Bestrahlen von mindestens einem Teil der Implantatoberfläche mit UV von 100 bis 315 nm während eines Zeitraums, der von 30 Sekunden bis 30 Minuten reicht und wahlweise
        c) das Waschen des Implantats,

wobei die Schritte a) und b) gleichzeitig oder in beliebiger Reihenfolge durchgeführt werden.

2. Das beschichtete Metallimplantat nach Anspruch 1, wobei der Schritt b) gleichzeitig mit Schritt a) durchgeführt wird oder ersatzweise der Schritt a) vor dem Schritt b) durchgeführt wird.

3. Das beschichtete Metallimplantat nach Ansprüchen 1 oder 2, wobei das UV-Licht des Schritts (b) ca. 6 mW/cm$^2$ beträgt.

4. Das beschichtete Metallimplantat nach einem der vorhergehenden Ansprüche, wobei das Implantat mit PUFA bei einer Konzentration beschichtet ist, die von 1 bis 10 Nanogramm/mm$^2$ reicht.

**5.** Das beschichtete Metallimplantat nach einem der vorhergehenden Ansprüche, wobei das Metall ausgewählt ist aus der Gruppe bestehend aus Titan, Titanlegierung, Zirconium, Hafnium, Tantal, Niob, und Mischungen aus zwei oder mehreren von Zirconium, Hafnium, Tantal und Niob.

**6.** Das beschichtete Metallimplantat nach Anspruch 5, wobei das Implantat mindestens 90 Gew.-% Titan und/oder einer Titanlegierung umfasst.

**7.** Das beschichtete Metallimplantat nach einem der vorhergehenden Ansprüche, wobei die PUFA ausgewählt ist aus der Gruppe bestehend aus n-3 und n-6-Fettsäuren.

**8.** Das beschichtete Metallimplantat nach einem der vorhergehenden Ansprüche, wobei die PUFA ausgewählt ist aus der Gruppe bestehend aus Eicosapentaensäure, Arachidonsäure, Docosahexaensäure und Mischungen davon.

**9.** Das beschichtete Metallimplantat nach einem der vorhergehenden Ansprüche, wobei die PUFA EPA (Eicosapentaensäure) umfasst oder ist.

**10.** Das beschichtete Metallimplantat nach einem der vorhergehenden Ansprüche, wobei die Implantatbeschichtung weiterhin fettlösliche Vitamine, wie etwa Vitamin E umfasst.

**11.** Das beschichtete Metallimplantat nach einem der vorhergehenden Ansprüche, wobei das Implantat weiterhin 7-Dehydrocholesterol umfasst.

**12.** Das beschichtete Metallimplantat nach einem der vorhergehenden Ansprüche, wobei, wenn das Implantat mindestens 90 Gew.-% Titan und/oder einer Titanlegierung umfasst, die PUFA EPA (Eicosapentaensäure) umfasst oder ist, die Beschichtung von 1 bis $5 \times 10^{15}$ gebundenen ungesättigten Bindungen von EPA per $mm^2$ Titanoberfläche umfasst.

**Revendications**

**1.** Un implant de métal recouvert pour l'adhésion contrôlée de tissu minéralisé et/ou dur, dans lequel au moins une partie de la surface de l'implant est recouverte avec des AGPI (acides gras polyinsaturés) liés chimiquement par UV ayant une concentration de 10 nanogrammes/$mm^2$, ou moins, dans lequel l'implant de métal recouvert peut être obtenu moyennant un procédé comprenant :

    i) polir miroir l'implant,
    ii) laver,
    iii) passer à l'autoclave,

        a) traiter l'implant avec une solution comprenant AGPI, et
        b) irradier au moins une partie de la surface de l'implant à l'UV de 100 à 315 nm pendant une période de temps comprise de 30 seconds à 30 minutes et facultativement
        c) laver ledit implant,

dans lequel l'étape a) et b) sont effectuées de façon simultanée ou dans un ordre quelconque.

**2.** L'implant de métal recouvert selon la revendication 1, dans lequel l'étape b) est effectuée en même temps que l'étape a) ou alternativement l'étape a) est effectuée avant l'étape b).

**3.** L'implant de métal recouvert selon les revendications 1 ou 2, dans lequel la lumière UV de l'étape (b) est d'environ 6 mW/$cm^2$.

**4.** L'implant de métal recouvert selon l'une quelconque des revendications précédentes, dans lequel l'implant est recouvert avec AGPI ayant une concentration comprise de 1 à 10 nanogrammes/$mm^2$.

**5.** L'implant de métal recouvert selon l'une quelconque des revendications précédentes, dans lequel le métal est choisi dans le groupe constitué de titane, alliage de titane, zirconium, hafnium, tantale, niobium, et des mélanges de deux ou plus parmi le zirconium, le hafnium, le tantale et le niobium.

**6.** L'implant de métal recouvert selon la revendication 5, dans lequel l'implant comprend au moins 90% en poids de titane et/ou d'un alliage de titane.

**7.** L'implant de métal recouvert selon l'une quelconque des revendications précédentes, dans lequel l'AGPI est choisi dans le groupe constitué des acides gras n-3 et n-6.

**8.** L'implant de métal recouvert selon l'une quelconque des revendications précédentes, dans lequel l'AGPI est choisi dans le groupe constitué de l'acide eicosapentaénoïque, l'acide arachidonique, l'acide docosahexaénoïque et des mélanges de ceux-ci.

**9.** L'implant de métal recouvert selon l'une quelconque des revendications précédentes, dans lequel l'AGPI comprend ou est l'EPA (acide eicosapentaénoïque).

**10.** L'implant de métal recouvert selon l'une quelconque des revendications précédentes, dans lequel le recouvrement de l'implant comprend en outre des vitamines liposolubles, telles que la vitamine E.

**11.** L'implant de métal recouvert selon l'une quelconque des revendications précédentes, dans lequel l'implant comprend en outre 7-déshydrocholestérol.

**12.** L'implant de métal recouvert selon l'une quelconque des revendications précédentes, dans lequel, lorsque l'implant comprend au moins 90% en poids de titane et/ou d'un alliage de titane, l'AGPI comprend ou est l'EPA (acide eicosapentaénoïque), alors le recouvrement comprend de 1 à $5 \times 10^{15}$ de liaisons insaturées liées d'EPA par $mm^2$ de surface de titane.

1. Preparation

Polishing
Washing
Autoclaving

2. Addition of
EPA in methanol

| Control | | | Control | | | Control | | |
|---|---|---|---|---|---|---|---|---|
| 0 µmol | 0.1µmol | 0.5µmol | 0 µmol | 0.1µmol | 0.5µmol | 0 µmol | 0.1µmol | 0.5µmol |

3. UV irradiation
for 30 min

UV irradiation

4. Washing in
methanol

2 x washing

2 x washing

Group

A

B

C

**Fig. 1**

**Fig. 2**

**Fig. 3**

Fig. 4

Fig. 5

**Fig. 6**

Fig. 7

**Fig. 8**

**Fig. 9**

Fig. 10

**Fig. 11**

Fig. 12

Fig. 13

Fig. 14

Fig. 15

Fig. 16

Fig. 17

Fig. 18

Fig. 19

Fig. 20

Fig. 21

Fig. 22

**7-DHC**

1%  *UV light*
**Skin**  ①

②  →  Lumisterol
         Tachysterol

**Previtamin D$_3$**  ③  →  Previtamin D$_3$ octanate
                              Previtamin D$_3$ decanoate

80%  *Heat*  ↑ ①
     **Skin**

②  Suprasterol I
③  Suprasterol II  }→ Ergosterols
    5,6-transvitamin D$_3$

**Cholecalciferol**  →  Vitamin D$_3$ octanate
                          Vitamin D$_3$ decanoate

*CYP27A1*
**Liver, bone**  ④

**25(OH)D$_3$**

*Hydroxylase*
*enzyme*  ⑤⑥
**Kidney, bone**

**1,25(OH)$_2$D$_3$**

**Fig. 23**

Fig. 24

Fig. 25

Fig. 26

**Fig. 27**

Fig. 28

Fig. 29

# EP 2 310 059 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2006088596 A
- US 2007202149 A
- US 2008118544 A
- US 2007299512 A
- US 61056978 B

### Non-patent literature cited in the description

- **GUILLÉN MD ; CABO N.** Infrared Spectroscopy in the Study of Edible Oils and Fats. *Journal of the Science of Food and Agriculture,* 1997, vol. 75, 1-11
- **KIM HK ; KIM K ; BYUN Y.** Preparation of a chemically anchored phospholipid monolayer on an acrylated polymer substrate. *Biomaterials,* 2005, vol. 26 (17), 3435-3444
- **NAKAMURA M ; SIRGHI L ; AOKI T ; HATANAKA Y.** Study on hydrophilic property of hydro-oxygenated amorphous TiOx:OH thin films. *Surface Science,* 2002, vol. 507-510, 778-782
- **RØNOLD HJ ; ELLINGSEN JE.** The use of a coin shaped implant for direct in situ measurement of attachment strength for osseointegrating biomaterial surfaces. *Biomaterials,* 2002, vol. 23 (10), 2201-2209
- **SATSANGI N ; SATSANGI A ; GLOVER R ; ONG JL ; SATSANGI RK.** Osteoblast response and calcium deposition on phospholipid modified surfaces. *J Mater Sci Mater Med,* 2004, vol. 15 (6), 693-7
- **MIYAUCHI M ; WATANABE T ; HASHIMOTO K ; KIEDA N ; HISHITA S ; MITSUHASHI T ; NAKAJIMA A.** Reversible wettability control of TiO2 surface by light irradiation. *Surface Science,* 2002, vol. 511, 401-407
- **ZHAO G ; SCHWARTZ Z ; WIELAND M ; GEIS-GERSTORFER J ; COCHRAN DL ; BOYAN BD.** High surface energy enhances cell response to titanium substrate microstructure. *Journal of Biomedical Materials Research,* 2005, vol. 74A (1), 49-58
- **WANG R ; HASHIMOTO K ; FUJISHIMA A ; CHIKUNI M ; KOJIMA E ; KITAMURA A ; SHIMOHIGOSHI M ; WATANABE T.** Photogeneration of Highly Amphiphilic TiO2 Surfaces. *Advanced Materials,* 1998, vol. 10 (2), 135-138

- **MOISON RM ; STEENVOORDEN DP ; BEIJERSBERGEN VAN HENEGOUWEN GM.** Topically applied eicosapentaenoic acid protects against local immunosuppression induced by UVB irradiation, cis-urocanic acid and thymidine dinucleotides. *Photochem Photobiol,* 2001, vol. 73 (1), 64-70
- **KHAN W ; KAPOOR M ; KUMAR N.** Covalent attachment of proteins to functionalized polypyrrole-coated metallic surfaces for improved biocompatibility. *Acta Biomaterialia,* 2007, vol. 3 (4), 541-549
- **MULLER R ; ABKE J ; SCHNELL E ; SCHARNWEBER D ; KUJAT R ; ENGLERT C ; TAHERI D ; NERLICH M ; ANGELE P.** Influence of surface pretreatment of titanium- and cobalt-based biomaterials on covalent immobilization of fibrillar collagen. *Biomaterials,* 2006, vol. 27 (22), 4059-4068
- **COOPER SL ; TEGOULIA VA ; RAO W ; KALAMBUR AT ; RABOLT JF.** Surface Properties, Fibrinogen Adsorption, and Cellular Interactions of a Novel Phosphorylcholine-Containing Self-Assembled Monolayer on Gold. *Langmuir,* 2001, vol. 17, 4396-4404
- **SVEDHEM S ; DAHLBORG D ; EKEROTH J ; KELLY J ; HOOK F ; GOLD J.** In Situ Peptide-Modified Supported Lipid Bilayers for Controlled Cell Attachment. *Langmuir,* 2003, vol. 19, 6730-6736
- **IWASAKI Y ; TOJO Y ; KUROSAKI T ; NAKABAYASHI N.** Reduced adhesion of blood cells to biodegradable polymers by introducing phosphorylcholine moieties. *J Biomed Mater Res A,* 2003, vol. 65 (2), 164-169
- **BRUNETTE DM ; TENGVALL P ; TEXTOR M ; THOMSON P.** Titanium in Medicine. Springer-Verlag, 2001
- **HIRANI N ; CHVEDOV D ; JONES R.** Characterization of organic monolayers on the surface of aluminum in the process of thermal treatment. *Thin Solid Films,* 2007
- **SOCRATES G.** Infrared and Raman Characteristic Group Frequencies. John Wiley & Sons Ltd, 2001

- **CHENG H ; JIANG, W. ; PHILLIPS, F.M. ; HAYDON, R.C. ; PENG, Y. ; ZHOU, L. ; LUU, H.H. ; AN, N. ; BREYER, B. ; VANICHAKARN, P.** Osteogenic Activity of the Fourteen Types of Human Bone Morphogenetic Proteins (BMPs). *The Journal of Bone and Joint Surgery,* 2003, vol. 85, 1544-1552
- **MELØ TB ; MAHMOUD GS.** Radicals Induced by Illumination of a Mixture of Unsaturated Fatty Acids with Ultraviolet Light at 77K. *Magnetic Resonance in Chemistry,* 1988, vol. 26, 947-954
- **CHACON JN ; GAGGINI P ; SINCLAIR RS ; SMITH FJ.** Photo- and thermal-oxidation studies on methyl and phenyl linoleate: anti-oxidant behaviour and rates of reaction. *Chemistry and Physics of Lipids,* 2000, vol. 107 (1), 107-120
- **RONTANI JF.** Photodegradation of unsaturated fatty acids in senescent cells of phytoplankton: photoproduct structural identification and mechanistic aspects. *Journal of Photochemistry and Photobiology A: Chemistry,* 1998, vol. 114 (1), 37-44
- **REFSGAARD HH ; TSAI L ; STADTMAN ER.** Modifications of proteins by polyunsaturated fatty acid peroxidation products. *Proc Natl Acad Sci U S A,* 2000, vol. 97 (2), 611-616
- **KIM B-H ; LEE J-Y ; CHOA Y-H ; HIGUCHI M ; MIZUTANI N.** Preparation of TiO2 thin film by liquid sprayed mist CVD method. *Materials Science and Engineering B,* 2004, vol. 107 (3), 289-294
- **WILLUMEIT R ; SCHUSTER A ; ILIEV P ; LINSER S ; FEYERABEND F.** Phospholipids as implant coatings. *J Mater Sci Mater Med,* 2007, vol. 18 (2), 367-380
- **CHEUNG SC ; SZETO YT ; BENZIE IF.** Antioxidant protection of edible oils. *Plant Foods Hum Nutr,* 2007, vol. 62 (1), 39-42
- **CAPDEVILA JH ; FALCK JR ; HARRIS RC.** *Cytochrome P450 and arachidonic acid bioactivation: molecular and functional properties of the arachidonate monooxygenase,* 2000, vol. 41, 163-18
- **WILLIAMS DL ; MARKS V.** Biochemistry in Clinical Practice. William Heineman Medical Books, 1983
- **HAN, Y ; CHEN, D ; SUN, J ; ZHANG, Y ; XU, K.** UV-enhanced bioactivity and cell response of micro-arc oxidized titania coatings. *Acta Biomater,* 2008, vol. 4, 1518-1529
- **VIORNERY, C et al.** Surface Modification of Titanium with Phosphonic Acid To Improve Bone Bonding: . *Characterization by XPS and ToF-SIMS,* 2002, vol. 18, 2582-2589
- **LU, G ; BERNASEK, SL ; SCHWARTZ, J.** Oxidation of a polycrystalline titanium surface by oxygen and water. *Surface Science,* 2000, vol. 458, 80-90
- **BEAMSON, G ; BRIGGS, D.** High resolution XPS of organic polymers : the Scienta ESCA300. John Wiley & Sons, 1992
- **RENO F ; AINA V ; GATTIA S ; CANNAS M.** Effect of vitamin E addition to poly(D,L) lactic acid on surface properties and osteoblast behaviour. *Biomaterials,* 2005, vol. 26, 5594-5599
- **WANG X ; QUINN PJ.** Vitamin E and its function in membranes. *Progress in Lipid Research,* 1999, vol. 38, 309-336
- **YAMAUCHI R ; NORO H ; SHIMOYAMADA M ; KATO K.** Analysis of Vitamin E and Its Oxidation Products by HPLC with Electrochemical Detection. *Lipids,* 2002, vol. 37 (5), 515-522
- **AIBA I ; YAMASAKI T ; SHINKI T ; IZUMI S ; YAMAMOTO K ; YAMADA S ; TERATO H ; IDE H ; OHYAMA Y.** Characterization of rat and human CYP2J enzymes as Vitamin D 25-hydroxylases. *Steroids,* 2006, vol. 71, 849-856
- **ATKINS GJ ; ANDERSON PH ; FINDLAY DM ; WELLDON KJ ; VINCENT C ; ZANNETTINO ACW ; O'LOUGHLIN PD ; MORRIS HA.** Metabolism of vitamin D3 in human osteoblasts: Evidence for autocrine and paracrine activities of $1\alpha,25$-dihydroxyvitamin D3. *Bone,* 2007, vol. 40, 1517-1528
- Degradation of vitamin D3 in a stressed formulation: The identification of esters of vitamin D3 formed by a transesterification with triglycerides. **BALLARD JM ; ZHU L ; NELSON ED ; SEBURG RA.** Journal of Pharmaceutical and Biomedical Analysis 43, 142-150, 2007 Holick MF, Photobiology of Vitamin D in Vitamin D. Elsevier, 2005
- **HOLICK MF.** Photobiology of Vitamin D in Vitamin D. Elsevier, 2005
- **ICHIKAWA F ; SATO K ; NANJO M ; NISHII Y ; SHINKI T ; TAKAHASHI N ; SUDA T.** Mouse Primary Osteoblasts Express Vitamin D3, 25-Hydroxylase mRNA and Convert $1\alpha$-Hydroxyvitamin D3, into $1\alpha,25$-Dihydroxyvitamin D3. *Bone,* 1995, vol. 16, 129-135
- **OLDS WJ ; MCKINLEY AR ; MOORE MR ; KIMLIN MG.** In vitro model of vitamin D3 (Cholecalciferol) synthesis by UV radiation: Dose-response relationships. *Journal of Photochemistry and Photobiology B: Biology,* 2008, vol. 93, 88-93
- **ST-ARNAUD R.** The direct role of vitamin D on bone homeostasis. *Archives of Biochemistry and Biophysics,* 2008, vol. 473, 225-230
- **S.-M. OH et al.** *Thin Solid Films,* 2003, vol. 435, 252-258

SEQUENCE LISTING

<110> NUMAT AS

<120> PUFA covered implants

<130> PD53788PC00

<150> US61/056,978
<151> 2008-05-29

<160> 14

<170> PatentIn version 3.3

<210> 1
<211> 20
<212> DNA
<213> Artificial

<220>
<223> Primer

<400> 1
gtaacccgtt gaaccccatt          20

<210> 2
<211> 20
<212> DNA
<213> Artificial

<220>
<223> Primer

<400> 2
ccatccaatc ggtagtagcg          20

<210> 3
<211> 20
<212> DNA
<213> Artificial

<220>
<223> Primer

<400> 3
acccagaaga ctgtggatgg          20

<210> 4
<211> 20
<212> DNA
<213> Artificial

<220>
<223> Primer

<400> 4
cacattgggg gtaggaacac          20

<210> 5
<211> 20
<212> DNA
<213> Artificial

<220>
<223> Primer

<400> 5
agagcatgac cgatggattc          20

<210> 6
<211> 20
<212> DNA

<213> Artificial

<220>
<223> Primer

<400> 6
ccttcttgag gttgccagtc        20

<210> 7
<211> 20
<212> DNA
<213> Artificial

<220>
<223> Primer

<400> 7
actggctagg tggtggtcag        20

<210> 8
<211> 20
<212> DNA
<213> Artificial

<220>
<223> Primer

<400> 8
ggtagggagc tgggttaagg        20

<210> 9
<211> 20
<212> DNA
<213> Artificial

<220>
<223> Primer

<400> 9
aacccagaca caagcattcc        20

<210> 10
<211> 20
<212> DNA
<213> Artificial

<220>
<223> Primer

<400> 10
gagagcgaag ggtcagtcag        20

<210> 11
<211> 17
<212> DNA
<213> Artificial

<220>

<223> Primer

<400> 11
ccgggagcag tgtgagc          17

<210> 12
<211> 21
<212> DNA
<213> Artificial

<220>
<223> Primer

<400> 12
tagatgcgtt tgtaggcggt c          21

<210> 13
<211> 20
<212> DNA
<213> Artificial

<220>
<223> Primer

<400> 13
gaaaatggag acggcgatag          20

<210> 14
<211> 20
<212> DNA
<213> Artificial

<220>
<223> Primer

<400> 14
acccgagagt gtggaaagtg          20